# EUROPEAN PATENT SPECIFICATION

(11) **EP 3 075 312 B1**
(45) Date of publication and mention of the grant of the patent: **29.07.2020**
(21) Application number: 15161804.8
(22) Date of filing: 31.03.2015
(51) Int. Cl.: A61B 5/053, A61B 5/16, A61B 5/00

(54) **Apparatuses for measuring skin resistance**
Vorrichtungen zur Messung des Hautwiderstands
Appareils pour mesurer la résistance de la peau

(43) Date of publication of application: 05.10.2016
(73) Proprietor: Nokia Technologies Oy, 02610 Espoo (FI)
(72) Inventor: Seko, Shigeyuki, Campbell, CA California 95008 (US)
(74) Representative: Whiting, Gary

(56) References cited:
- WO-A1-2013/149181
- WO-A1-2014/138414
- WO-A2-2008/099288
- US-A1- 2008 081 963

## Description

### TECHNICAL FIELD

The present application relates generally to apparatuses and methods for measuring skin resistance.

### BACKGROUND

As electronic apparatuses become increasingly prevalent and pervasive in our society, people increasingly utilize electronic apparatuses to measure values. Many users may rely on electronic apparatuses for purposes relating to measuring particular values. As such, it may be desirable to configure an electronic apparatus such that the electronic apparatus facilitates measuring particular values in an intuitive and simple manner.

WO 2008/1099288 states that one aspect of the invention disclosed therein relates to a portable, handheld biosensor device that is held between two fingers of the same hand or otherwise contacts two points on a user's skin. The sensor device includes a pair of conductive or semi- conductive electrodes and associated circuitry designed to sense, amplify and digitize the electrical conductance of the skin between the electrodes. The device can additionally be configured to sense additional biometrics from the fingers, including blood oxygenation and skin temperature. Digitized biometric values are transmitted wirelessly (or via direct wire connection, such as a USB cable) to a computing device where the data is utilized to generate a control parameter in a software application whose purpose is to provide anxiety biofeedback or entertainment.

US 2008/0081963 discloses methods and an apparatus for determining individual responses to induced and actual stress and for identifying individuals susceptible to detrimental effects of mental stress on the cardiovascular system. The subjects may be at risk for chronic effects of mental stress by virtue of synergistic effects of mental stress and cardiovascular (CV) disease risk factors) or may be at risk, or vulnerable to, acute effects of mental stress by virtue of synergistic effects of mental stress and underlying hidden coronary atherosclerosis. The invention further provides an apparatus for assessing vascular reactivity in individuals under ambulatory conditions. Document WO 2013/149181 (A1) is also pertinent to understand the background of the invention.

### SUMMARY

Various aspects of example embodiments are set out in the summary, the drawings, the detailed description, and the claims.

One or more example embodiments may provide an apparatus, a computer readable medium, a non-transitory computer readable medium, a computer program product, and/or a method for receiving, from a circuit, information indicative of a skin resistance between an inner electrode and an outer electrode, the inner electrode being disposed on at least part of an inner surface of a housing, the outer electrode being disposed on at least part of an outer surface of the housing such that the outer electrode is separated from the inner electrode by, for example, space, an electrically insulating material, or another means, the housing being configured to be in contact with or at least partially encircle a body part of a user of the housing, such that the inner surface of the housing is configured to at least partially contact the body part and the outer surface of the housing is configured to avoid contact with the body part, and determining a galvanic skin response of the user based, at least in part, on the skin resistance when the outer electrode is in contact with the user.

One or more example embodiments may provide an apparatus, a computer readable medium, a computer program product, and/or a non-transitory computer readable medium having means for receiving, from a circuit, information indicative of a skin resistance between an inner electrode and an outer electrode, the inner electrode being disposed on at least part of an inner surface of a housing, the outer electrode being disposed on at least part of an outer surface of the housing such that the outer electrode is separated from the inner electrode (e.g., by an electrically insulating material), the housing being configured to be in contact with or at least partially encircle a body part of a user of the housing, such that the inner surface of the housing is configured to at least partially contact the body part and the outer surface of the housing is configured to avoid contact with the body part, and means for determining a galvanic skin response of the user based, at least in part, on the skin resistance when the outer electrode is in contact with the user.

Some implementations include an apparatus comprising a housing that comprises an inner surface and an outer surface; an inner electrode disposed on at least part of the inner surface; an outer electrode disposed on at least part of the outer surface such that the outer electrode is electrically separated from the inner electrode; and at least one circuit configured to measure skin resistance between the inner electrode and the outer electrode when the inner electrode and the outer electrode are in contact with a user.

In at least one example embodiment, the inner electrode and the outer electrode are configured to form a shortest electrical path through the user, and the shortest electrical path is longer than any path through the housing between the inner electrode and the outer electrode.

Some implementations include an apparatus comprising a housing that is configured to be in contact with or at least partially encircle a body part of a user of the apparatus, such that an inner surface of the housing is configured to at least partially contact the body part and an outer surface of the housing is configured to avoid contact with the body part, an inner electrode disposed on at least part of the inner surface of the housing, an outer electrode disposed on at least part of the outer surface of the housing such that the outer electrode is separated from the inner electrode (e.g., by an electrically insulating material), and at least one circuit configured to measure skin resistance between the inner electrode and the outer electrode.

In some implementations, the housing may include or use an interface module configured to communicate with a separate device or apparatus. The interface module may send information of the skin resistance to the separate device or apparatus and/or receive information from the separate device or apparatus.

In at least one example embodiment, the body part is at least one of a finger, a wrist, an ankle, a neck, a waist, a head, a limb, and/or a toe.

In at least one example embodiment, the housing is in a form configured to at least partially encircle a part of the user, with the inner surface configured to contact the body part of the user. For instance, in at least one example embodiment, the housing is a ring configured to at least partially encircle a finger of the user.

In at least one example embodiment, the housing is a bracelet configured to at least partially encircle a wrist of the user.

In at least one example embodiment, the housing is at least partially toroidal.

In at least one example embodiment, the housing is a continuously toroidal shape that is configured to completely encircle the body part.

In at least one example embodiment, the housing is a discontinuously toroidal shape that is configured to partially encircle the body part without completely encircling the body part.

In at least one example embodiment, the housing comprises a gap in the toroidal shape such that the toroidal shape is the discontinuously toroidal shape.

In at least one example embodiment, the housing is at least a part of a watch, bracelet, or ring, the housing further comprising an indication device configured to provide, based on the skin resistance, a visual indicator, an audio indicator, or both.

In at least one example embodiment, the housing comprises at least one outward protrusion from the outer surface of the housing.

In at least one example embodiment, the outward protrusion comprises at least one display element.

In at least one example embodiment, the outer surface of the housing comprises at least one channel that is configured to partially encircle a different body part of the user. The channel is configured to, for example, provide an increased contact area.

In at least one example embodiment, the different body part is a finger and the channel is shaped in correspondence with a shape of a finger.

In at least one example embodiment, the housing is a ring configured to at least partially encircle a different finger of the user and the channel is sized substantially the same as the inner surface of the housing.

In at least one example embodiment, at least part of the inner electrode is concentric with at least part of the outer electrode.

In at least one example embodiment, the electrically insulating material is concentric with the inner electrode and the outer electrode.

In at least one example embodiment, the electrically insulating material forms a concentric separation between the inner electrode and the outer electrode.

In at least one example embodiment, the inner electrode is disposed longitudinally on the inner surface of the housing.

In at least one example embodiment, the inner electrode is longitudinally contiguous.

In at least one example embodiment, the inner electrode is disposed on at least part of the inner surface of the housing exclusive of disposition on the outer surface of the housing.

In at least one example embodiment, the inner electrode fails to be disposed on any part of the outer surface of the housing.

In at least one example embodiment, the outer electrode is disposed on at least part of the outer surface of the housing exclusive of disposition on the inner surface of the housing.

In at least one example embodiment, the outer electrode fails to be disposed on any part of the inner surface of the housing.

In at least one example embodiment, the outer electrode comprises a radial gap that is electrically insulated from the outer electrode and the inner electrode.

In at least one example embodiment, the housing further comprises a tactile indicator disposed within the radial gap that tactilely differentiates the radial gap from the outer electrode.

In at least one example embodiment, an electrode is an electrical conductor configured to electrically couple skin of the user with the circuit.

In at least one example embodiment, the electrode is configured to contact skin of the user.

In at least one example embodiment, comprising a wireless transmitter coupled with the housing configured to wirelessly transmit information indicative of the skin resistance between the inner electrode and the outer electrode.

In at least one example embodiment, the apparatus further comprises at least one processor and at least one memory, the memory comprising machine-readable instructions, that when executed cause the apparatus to perform receipt, from the circuit, of information indicative of the skin resistance between the inner electrode and the outer electrode, and determination of a galvanic skin response of the user based, at least in part, on the skin resistance.

In at least one example embodiment, determination of the galvanic skin response is based, at least in part, on variation in the skin resistance over time.

In at least one example embodiment, determination of the galvanic skin response is based, at least in part, on a difference between the skin resistance from a predetermined skin resistance value.

One or more example embodiments further perform determination that the skin resistance is within a skin contact threshold value,

In at least one example embodiment, determination of the galvanic skin response is performed in response to the determination that the skin resistance is within the skin contact threshold value.

One or more example embodiments further perform determination that the skin resistance has changed to be beyond the skin contact threshold value, and preclusion of determination of a galvanic skin response of the user in response to the determination that the skin resistance has changed to be beyond the skin contact threshold value.

One or more example embodiments further perform establishment of a communication channel with a separate apparatus, and causation of sending information indicative of the galvanic skin response to the separate apparatus by way of the communication channel.

One or more example embodiments further comprise a separate housing that is configured to at least partially encircle a different body part of the user such that an inner surface of the separate housing is configured to at least partially contact a body part, the inner surface of the separate housing is electrically connected to an outer surface of the separate housing, which is configured to contact the outer electrode of the housing, providing the contact of the outer electrode of the housing with the user.

In at least one example embodiment, the outer surface of the housing comprises at least one channel that is configured to partially encircle the separate housing.

In at least one example embodiment, the channel is sized substantially the same as the outer surface of the separate housing.

In at least one example embodiment, the separate housing consists entirely of electrically conductive material.

In at least one example embodiment, the outer surface of the separate housing comprises an electrically conductive surface and an electrically insulated surface, the electrically conductive surface being a portion of the outer surface of the separate housing that comprises an electrically conductive material and the electrically insulating surface being a portion of the outer surface of the separate housing that comprises an electrically insulating material.

In at least one example embodiment, the electrically insulating surface of the separate housing further comprises a tactile indicator that tactilely differentiates the electrically insulating surface of the separate housing from the electrically conductive surface of the separate housing.

In at least one example embodiment, the outer surface of the separate housing comprises at least one channel that is configured to partially encircle the housing.

In at least one example embodiment, the channel is sized substantially the same as the outer surface of the housing.

### BRIEF DESCRIPTION OF THE DRAWINGS

For a more complete understanding of one or more example embodiments, reference is now made to the following descriptions taken in connection with the accompanying drawings in which:
FIG. 1 is a block diagram showing an apparatus according to at least one example embodiment;
FIGS. 2A-2C are diagrams illustrating apparatuses according to at least one example embodiment;
FIGS. 3A-3D are diagrams illustrating skin resistance measurement according to at least one example embodiment;
FIGS. 4A-4J are diagrams illustrating housings according to at least one example embodiment;
FIGS. 5A-5E are diagrams illustrating separate housings according to at least one example embodiment;
FIGS. 6A-6E are diagrams illustrating housings according to at least one example embodiment;
FIG. 7 is a flow diagram illustrating activities associated with determining a galvanic skin response according to at least one example embodiment;
FIG. 8 is a flow diagram illustrating activities associated with determining a skin contact threshold value according to at least one example embodiment; and
FIG. 9 is a flow diagram illustrating activities associated with sending information to a separate apparatus according to at least one example embodiment.

### DETAILED DESCRIPTION OF THE DRAWINGS

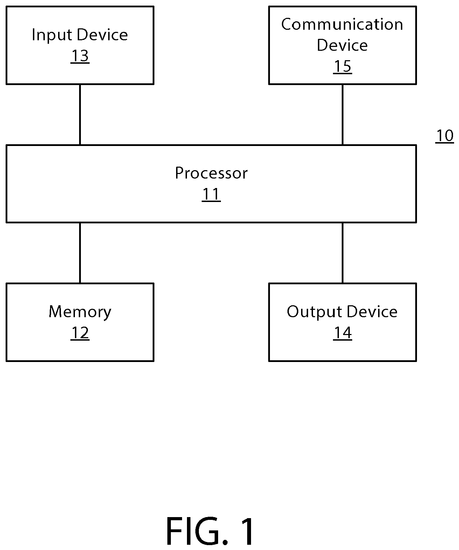

Various example embodiments and some of their potential advantages are understood by referring to FIGS. 1 through 9 of the drawings.

Some example embodiments will now further be described hereinafter with reference to the accompanying drawings, in which some, but not all, example embodiments are shown. One or more example embodiments may be embodied in many different forms and the claims should not be construed as being strictly limited to the example embodiments set forth herein; rather, these example embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with one or more example embodiments. Thus, use of any such terms should not be taken to limit the scope of the disclosure. The scope of protection is defined by the appended claims.

Additionally, as used herein, the term 'circuitry' refers to (a) hardware-only circuit implementations (e.g., implementations in analog circuitry, digital circuitry and/or any combination thereof); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that utilize software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, a baseband integrated circuit, an applications processor integrated circuit, a cellular network apparatus, other network apparatus, and/or other computing apparatus.

As defined herein, a "non-transitory computer readable medium," which refers to a physical medium (e.g., volatile or non-volatile memory device), can be differentiated from a "transitory computer-readable medium," which refers to an electromagnetic signal. In at least one example embodiment, a non-transitory computer readable medium is a tangible non-transitory computer readable medium.

FIG. 1 is a block diagram showing an apparatus, such as an electronic apparatus 10, according to at least one example embodiment. It should be understood, however, that an electronic apparatus as illustrated and hereinafter described is merely illustrative of an electronic apparatus that could benefit from one or more example embodiments. While electronic apparatus 10 is illustrated and will be hereinafter described for purposes of example, other types of electronic apparatuses may readily employ one or more example embodiments. Electronic apparatus 10 may be a personal digital assistant (PDAs), a pager, a mobile computer, a desktop computer, a television, a gaming apparatus, a laptop computer, a tablet computer, a media player, a camera, a video recorder, a mobile phone, a global positioning system (GPS) apparatus, an automobile, a kiosk, an electronic table, a wearable apparatus, a ring apparatus, a bracelet apparatus, a pendant apparatus, a necklace apparatus, a belt apparatus, a headband apparatus, an anklet apparatus, a near eye display, a head mounted display, any apparatus that is configured to determine a galvanic skin response of an individual, and/or any other types of electronic systems. Moreover, the apparatus of at least one example embodiment need not be the entire electronic apparatus, but may be a component or group of components of the electronic apparatus in other example embodiments. For example, the apparatus may be an integrated circuit, a set of integrated circuits, and/or the like.

Furthermore, apparatuses may readily employ one or more example embodiments regardless of any intent to provide mobility. In this regard, even though some example embodiments may be described in conjunction with mobile applications, it should be understood that such example embodiments may be utilized in conjunction with a variety of other applications, both in the mobile communications industries and outside of the mobile communications industries. For example, the apparatus may be a mobile or portable apparatus, or at least part of a non-carryable or non-portable apparatus, such as a large screen television, an electronic table, a kiosk, an automobile, and/or the like.

In at least one example embodiment, electronic apparatus 10 comprises at least one processor, such as processor 11 and at least one memory, such as memory 12. Processor 11 may be any type of processor, controller, embedded controller, processor core, and/or the like. In at least one example embodiment, processor 11 utilizes computer program code to cause an apparatus to perform one or more actions. Memory 12 may comprise volatile memory, such as volatile Random Access Memory (RAM) including a cache area for the temporary storage of data and/or other memory, for example, non-volatile memory, which may be embedded and/or may be removable. The non-volatile memory may comprise an EEPROM, flash memory and/or the like. Memory 12 may store any of a number of pieces of information, and data. The information and data may be used by the electronic apparatus 10 to implement one or more functions of the electronic apparatus 10, such as the functions described herein. In at least one example embodiment, memory 12 includes computer program code such that the memory and the computer program code are configured to, working with the processor, cause the apparatus to perform one or more actions described herein.

The electronic apparatus 10 may further comprise a communication device 15. In at least one example embodiment, communication device 15 comprises an antenna, (or multiple antennae), a wired connector, and/or the like in operable communication with a transmitter and/or a receiver. In at least one example embodiment, processor 11 provides signals to a transmitter and/or receives signals from a receiver. The signals may comprise signaling information in accordance with a communications interface standard, user speech, received data, user generated data, and/or the like. Communication device 15 may operate with one or more air interface standards, communication protocols, modulation types, and access types (e.g., one or more standards in the Institute of Electrical and Electronics Engineers (IEEE) 802 family of wired and wireless standards). By way of illustration, the electronic communication device 15 may operate in accordance with second-generation (2G) wireless communication protocols IS-136 (time division multiple access (TDMA)), Global System for Mobile communications (GSM), and IS-95 (code division multiple access (CDMA)), with third-generation (3G) wireless communication protocols, such as Universal Mobile Telecommunications System (UMTS), CDMA2000, wideband CDMA (WCDMA) and time division-synchronous CDMA (TD-SCDMA), and/or with fourth-generation (4G) wireless communication protocols, wireless networking protocols, such as 802.11, short-range wireless protocols, such as Bluetooth (e.g., Bluetooth 4.x or earlier or later), and/or the like. Communication device 15 may operate in accordance with wireline protocols, such as Ethernet, digital subscriber line (DSL), asynchronous transfer mode (ATM), and/or the like.

Processor 11 may comprise means, such as circuitry, for implementing audio, video, communication, navigation, logic functions, and/or the like, as well as for implementing one or more example embodiments including, for example, one or more of the functions described herein. For example, processor 11 may comprise means, such as a digital signal processor device, a microprocessor device, an analog to digital converter, a digital to analog converter, processing circuitry and other circuits, for performing various functions including, for example, one or more of the functions described herein. The apparatus may perform control and signal processing functions of the electronic apparatus 10 among these devices according to their respective capabilities. The processor 11 thus may comprise the functionality to encode and interleave message and data prior to modulation and transmission. The processor 1 may additionally comprise an internal voice coder, and may comprise an internal data modem. Further, the processor 11 may comprise functionality to operate one or more software programs, which may be stored in memory and which may, among other things, cause the processor 11 to implement at least one embodiment including, for example, one or more of the functions described herein. For example, the processor 11 may operate a connectivity program, such as a conventional internet browser. The connectivity program may allow the electronic apparatus 10 to transmit and receive internet content, such as location-based content and/or other web page content, according to a Transmission Control Protocol (TCP), Internet Protocol (IP), User Datagram Protocol (UDP), Internet Message Access Protocol (IMAP), Post Office Protocol (POP), Simple Mail Transfer Protocol (SMTP), Wireless Application Protocol (WAP), Hypertext Transfer Protocol (HTTP), and/or the like, for example.

The electronic apparatus 10 may comprise a user interface for providing output and/or receiving input. The electronic apparatus 10 may comprise an output device 14. Output device 14 may comprise an audio output device, such as a ringer, an earphone, a speaker, and/or the like. Output device 14 may comprise a tactile output device, such as a vibration transducer, an electronically deformable surface, an electronically deformable structure, and/or the like. Output device 14 may comprise a visual output device, such as a display, a light, and/or the like. In at least one example embodiment, the apparatus causes display of information, the causation of display may comprise displaying the information on a display comprised by the apparatus, sending the information to a separate apparatus, and/or the like. For example, the apparatus may send the information to a separate display, to a computer, to a laptop, to a mobile apparatus, and/or the like. For example, the apparatus may be a server that causes display of the information by way of sending the information to a client apparatus that displays the information. In this manner, causation of display of the information may comprise sending one or more messages to the separate apparatus that comprise the information, streaming the information to the separate apparatus, and/or the like. The electronic apparatus may comprise an input device 13. Input device 13 may comprise a light sensor, a proximity sensor, a microphone, a touch sensor, a force sensor, a button, a keypad, a motion sensor, a magnetic field sensor, a camera, and/or the like. A touch sensor and a display may be characterized as a touch display. In an embodiment comprising a touch display, the touch display may be configured to receive input from a single point of contact, multiple points of contact, and/or the like. In such an embodiment, the touch display and/or the processor may determine input based, at least in part, on position, motion, speed, contact area, and/or the like. In at least one example embodiment, the apparatus receives an indication of an input. The apparatus may receive the indication from a sensor, a driver, a separate apparatus, and/or the like. The information indicative of the input may comprise information that conveys information indicative of the input, indicative of an aspect of the input indicative of occurrence of the input, and/or the like.

The electronic apparatus 10 may include any of a variety of touch displays including those that are configured to enable touch recognition by any of resistive, capacitive, infrared, strain gauge, surface wave, optical imaging, dispersive signal technology, acoustic pulse recognition, or other techniques, and to then provide signals indicative of the location and other parameters associated with the touch. Additionally, the touch display may be configured to receive an indication of an input in the form of a touch event which may be defined as an actual physical contact between a selection object (e.g., a finger, stylus, pen, pencil, or other pointing device) and the touch display. Alternatively, a touch event may be defined as bringing the selection object in proximity to the touch display, hovering over a displayed object or approaching an object within a predefined distance, even though physical contact is not made with the touch display. As such, a touch input may comprise any input that is detected by a touch display including touch events that involve actual physical contact and touch events that do not involve physical contact but that are otherwise detected by the touch display, such as a result of the proximity of the selection object to the touch display. A touch display may be capable of receiving information associated with force applied to the touch screen in relation to the touch input. For example, the touch screen may differentiate between a heavy press touch input and a light press touch input. In at least one example embodiment, a display may display two-dimensional information, three-dimensional information and/or the like.

In example embodiments including a keypad, the keypad may comprise numeric (for example, 0-9) keys, symbol keys (for example, #, *), alphabetic keys, and/or the like for operating the electronic apparatus 10. For example, the keypad may comprise a conventional QWERTY keypad arrangement. The keypad may also comprise various soft keys with associated functions. In addition, or alternatively, the electronic apparatus 10 may comprise an interface device such as a joystick or other user input interface.

Input device 13 may comprise a media capturing element. The media capturing element may be any means for capturing an image, video, and/or audio for storage, display, or transmission. For example, in at least one example embodiment in which the media capturing element is a camera module, the camera module may comprise a digital camera which may form a digital image file from a captured image. As such, the camera module may comprise hardware, such as a lens or other optical component(s), and/or software for creating a digital image file from a captured image. Alternatively, the camera module may comprise only the hardware for viewing an image, while a memory device of the electronic apparatus 10 stores instructions for execution by the processor 11 in the form of software for creating a digital image file from a captured image. In at least one example embodiment, the camera module may further comprise a processing element that is separate from processor 11 for processing data, such as image data. The camera module may provide data, such as image data, in one or more of various formats. In at least one example embodiment, the camera module comprises an encoder, a decoder, and/or the like for compressing and/or decompressing image data. The encoder and/or decoder may encode and/or decode according to a standard format, for example, a Joint Photographic Experts Group (JPEG) standard format.

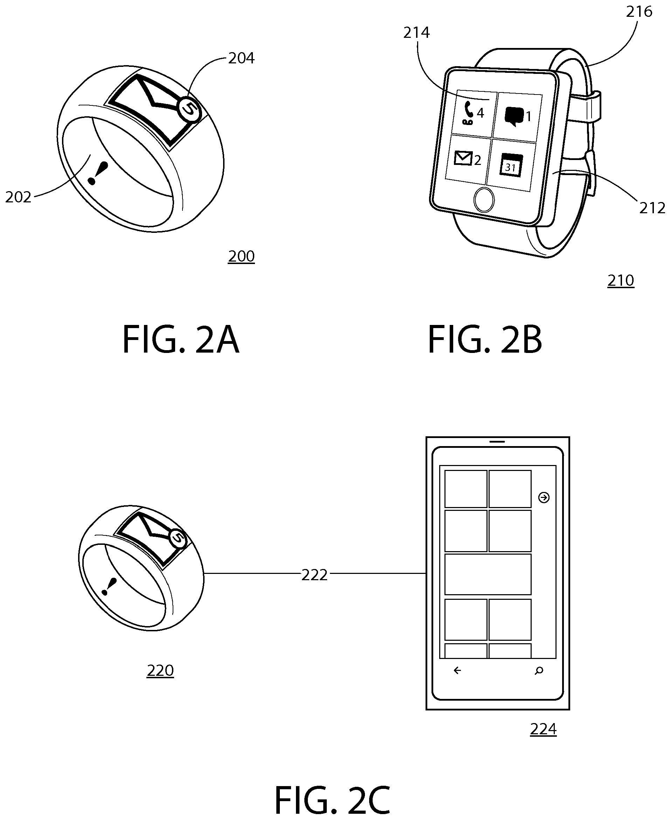

FIGS. 2A-2C are diagrams illustrating apparatuses according to at least one example embodiment. The examples of FIGS. 2A-2C are merely examples. For example, the configuration of the apparatuses may vary, the type of apparatuses may vary, the size of the apparatuses may vary, and/or the like.

In many circumstances, a user may desire to interact with an electronic apparatus throughout the user's day. For example, the user may desire to perceive indications associated with messages, notifications, emails, alerts, phone calls, and/or the like. In such an example, the user may desire to utilize the electronic apparatus in a manner that is easy and convenient for the user. For example, the user may desire to avoid holding the electronic apparatus in the user's hands, avoid storing the electronic apparatus in the user's pocket only to subsequently retrieve the electronic apparatus upon receipt of a notification, and/or the like. In circumstances such as these, it may be desirable for the apparatus to be a wearable apparatus. A wearable apparatus may refer to an apparatus that is configured to be worn by a user of the apparatus. For example, a wearable apparatus may be a ring apparatus, a bracelet apparatus, a necklace apparatus, and/or the like. A user wearing an apparatus may be referred to as a user of the apparatus, a wearer of the apparatus, and/or the like.

In some circumstances, for an apparatus to be wearable, it may be desirable for the apparatus to comprise a housing. For example, a housing may allow for the apparatus to be wearable, while keeping the overall size, form factor, and/or the like of the apparatus within a smaller footprint. For example, the housing of the apparatus may be configured to at least partially contact a body part of a user of the apparatus. For instance, the housing may partially contact the body part by at least partially encircling the body part. For example, the housing may be configured in the shape of a ring, a bracelet, a necklace, a pendant, and/or the like such that the apparatus may at least partially encircle a finger, a wrist, a neck, and/or the like of a user of the apparatus. Such a configuration of a housing may be referred to as a wearable housing. The housing may house additional components of the apparatus such that the bulk of the apparatus may be contained within the housing. In at least one example embodiment, an apparatus comprises a housing that is configured to at least partially contact a body part of a user of the apparatus.

FIG. 2A is a diagram illustrating at least a portion of apparatus 200. In the example of FIG. 2A, apparatus 200 comprises housing 202 and display 204. It can be seen in the example of FIG. 2A that housing 202 has a ring configuration. It should be understood that housing 202 is configured to at least partially encircle a finger of a user of housing 202. In this manner, it can be seen that housing 202 may be referred to as a wearable housing. For example, a user of apparatus 200 may wear housing 202 on a finger of the user such that display 204 may be viewed when housing 202 is worn. Even though the example of FIG. 2A is described as illustrating at least a portion of apparatus 200, it should be understood that, in some circumstances, FIG. 2A may illustrate the entirety of apparatus 200.

FIG. 2B is a diagram illustrating at least a portion of apparatus 210. In the example of FIG. 2B, apparatus 210 comprises housing 212, display 214, and adjustable wrist strap 216, which may be detached or replaced. It can be seen in the example of FIG. 2B that at least a portion of apparatus 210 has a bracelet configuration, a watch configuration, and/or the like. It should be understood that housing 212 is configured to at least partially encircle a wrist of a user of housing 212. In this manner, it can be seen that housing 212 may be referred to as a wearable housing. For example, a user of apparatus 210 may wear housing 212 on a wrist of the user such that display 214 may be viewed when housing 212 is worn. Even though the example of FIG. 2B is described as illustrating at least a portion of apparatus 210, it should be understood that, in some circumstances, FIG. 2B may illustrate the entirety of apparatus 210.

Even though the examples of FIGS. 2A and 2B illustrate a finger worn apparatus and a wrist worn apparatus, it should be understood that an apparatus may be configured to be worn on and/or used by other body parts of a user. For example, an apparatus may be configured to be used and/or worn around the neck, the waist, the head, an ankle, a limb, a toe, and/or the like of a user.

In many circumstances, a user may desire to communicate by way of an electronic apparatus. For example, the users may desire to send and/or receive messages, interact with other apparatuses, send and/or receive information, and/or the like. In order to facilitate such an experience, in many circumstances, it may be desirable to allow for communication between two or more apparatuses. For example, it may be desirable to allow for communication between an apparatus and a separate apparatus. In such an example, each of the apparatus and the separate apparatus may be a phone, a tablet, a computer, a laptop, an electronic apparatus, a server, a wearable apparatus, a head mounted apparatus, a projector, a near eye display, and/or the like. In at least one example embodiment, an apparatus and a separate apparatus communicate via a direct communication channel, an indirect communication channel, and/or the like. In such an example embodiment, the indirect communication channel may route communication between the apparatus and the separate apparatus by way of one or more routers, switches, hubs, distribution servers, networks, and/or the like. In at least one example embodiment, an apparatus and a separate apparatus communicate via an indirect communication channel, wireless and/or wired, by way of a server. In such an example embodiment, the server may be a computer, a service platform, a repository, an application, and/or the like. For example, the server, may be configured to update an account associated with the separate apparatus such that the separate apparatus may receive information from the apparatus by way of accessing the account via the server.

In the example of FIG. 2C, apparatus 220 may communicate with apparatus 224, directly or indirectly, by way of communication channel 222. For example, apparatus 220 may send information to apparatus 224, apparatus 220 may receive information sent from apparatus 224, and/or the like. It should be understood that, even though the example of FIG. 2C illustrates a direct communication channel between apparatus 220 and apparatus 224, there may be intermediate apparatuses that facilitate communication between apparatus 220 and apparatus 224. For example, there may be one or more routers, hubs, switches, gateways, base stations, networks, and/or the like, that are utilized in the communication channels between apparatus 220 and apparatus 224. Communication channel 222 may include any number of wired and/or wireless segments. In addition, there may be one or more separate apparatuses that apparatus 220 and/or apparatus 224 are in communication with. For example, apparatus 220 and/or apparatus 224 may be in communication with another apparatus, a separate apparatus, a different apparatus, and/or the like. Evan though the example of FIG. 2C illustrates apparatus 220 as a wearable apparatus, it should be understood that apparatus 220 may be any type of apparatus, such as a different configuration of a wearable apparatus, a non-wearable apparatus, a mobile device, and/or the like. Evan though the example of FIG. 2C illustrates apparatus 224 as a mobile device, it should be understood that apparatus 224 may be any type of apparatus, such as a wearable apparatus, a non-wearable apparatus, a non-mobile device, and/or the like.

In many circumstances, during communication with an apparatus and a separate apparatus, it may be desirable for the apparatuses to share information. For example, information shared between the apparatuses may help facilitate the communication, may provide useful information to the users of the apparatuses, and/or the like. Information shared between the apparatus and the separate apparatus may include information about the apparatus, information about the separate apparatus, information about the user of the apparatus, information about the user of the separate apparatus, and/or the like. For example, the apparatus and the separate apparatus may share information with respect to the location of the separate apparatus, a current operating condition of the separate apparatus, information captured by the apparatus, information recorded by the apparatus, information measured by the apparatus, and/or the like. In at least one example embodiment, the information shared between the apparatus and the separate apparatus comprises an image. For example, the apparatus and the separate apparatus may share an image that was captured by the apparatus and/or the separate apparatus similar as described regarding FIG. 1.

In some circumstances, a plurality of apparatuses may share information by way of local communication among the apparatuses. For example, the apparatuses may share information by way of low power radio frequency communication (e.g., Bluetooth® Low Energy in Bluetooth® 4.0, ANT+ communication protocol, etc.), a radio frequency communication, near field communication, inductive communication, electric field communication, Bluetooth communication, infrared communication, local area network communication, wireless local area network communication, local port communication, input/output port communication, and/or the like. In some circumstances, apparatuses may share information by way of non-local communication among the apparatuses. For example, the apparatuses may communicate by way of high power radio frequency communication, wide area network communication, internet communication, cellular network communication, and/or the like. In at least one example embodiment, an apparatus retains information associated with communication with a separate apparatus. For example, the apparatus may comprise information associated with identifying, communicating with, authenticating, performing authentication with, and/or the like, the separate apparatus. In this manner, the apparatus may be privileged to perform operations in conjunction with the separate apparatus that a different apparatus may lack the privilege to perform.

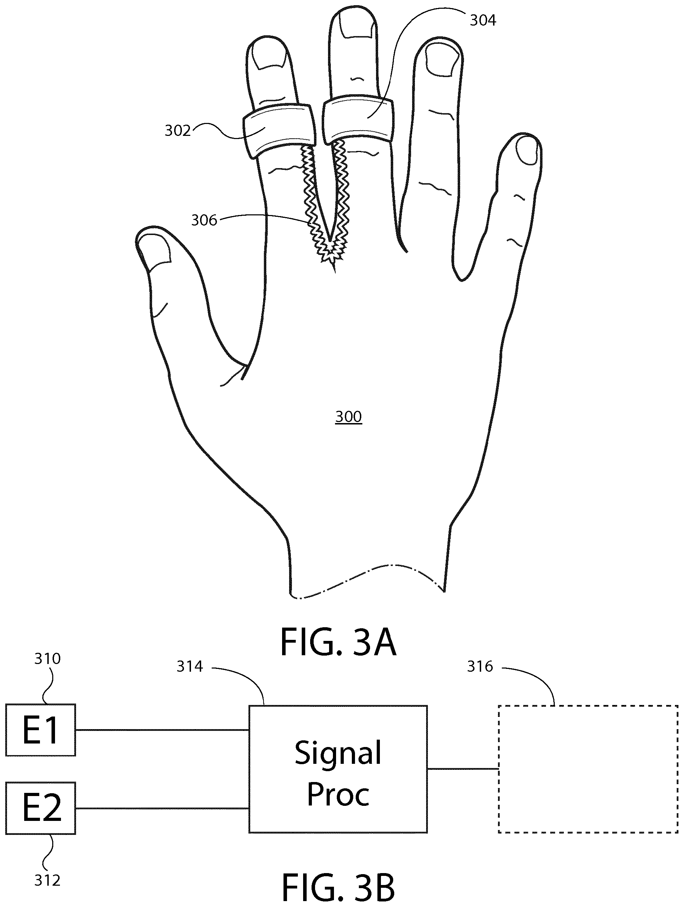

FIGS. 3A-3D are diagrams illustrating skin resistance measurement according to at least one example embodiment. The examples of FIGS. 3A-3D are merely examples. For example, the position of the electrodes may vary, the size of the electrodes may vary, the shape of the electrodes may vary, and/or the like.

It is known that an electrical conductor has a measurable resistance and a related measurable conductance that is the inverse of the resistance. Resistance refers the opposition of an electric conductor to an electrical current, whereas conductance refers to the ease with which an electrical current passes through a conductor. All materials have a measurable level of electrical resistance and a measurable level of conductance. For example, the electrical resistance of copper may be measured with electrical resistance measuring circuit, such as an ohmmeter. The subject matter described herein is applicable for measuring and using skin resistance and/or skin conductance. For simplicity, only skin resistance is described in some examples. It should be understood, however, that where applicable, the description with skin resistance is applicable to skin conductance and the like.

The body of an animal (e.g., a human body) is known to have a skin resistance. Skin resistance may refer to the opposition of skin to an electrical current. Similarly, the body is known to have a skin conductance that is the inverse to the skin resistance. Skin conductance may refer to the ease with which an electrical current may pass through the skin. Skin resistance and/or skin conductance may be measured. For example, the resistance of a portion of skin may be measured by placing a first electrode and a second electrode in contact with two different points on the skin, and measuring the resistance between the electrodes by way of a circuit. A circuit may be configured to measure skin resistance between a first electrode and a second electrode. Such a circuit may be referred to as a skin resistance measurement circuit. An electrode may refer to an electrical conductor configured to electrically couple or contact skin of the user with the circuit. It should be understood that an electrode may be different from an electrical connector. Generally, an electrode may be used to make contact with a non-metallic or non-conductive portion of a circuit (e.g. a portion of body skin), whereas an electrical connector will typically join two metallic portions of a circuit (e.g. a plug and a jack).

In the example of FIG. 3A, first electrode 302 is in contact with the skin of a finger or thumb (e.g., the index finger) of hand 300, and second electrode 304 is in contact with the skin of another finger (e.g., the middle finger) of hand 300 such that a skin resistance of hand 300 between first electrode 302 and second electrode 304 may be measured. For example, it should be understood that an electrical path 306 with a measurable skin resistance exists on and/or in the skin of hand 300 between first electrode 302 and second electrode 304. Even though first electrode 302 and second electrode 304 are illustrated with a ring configuration, it should be understood that first electrode 302 and second electrode 304 may be any configuration of electrode. Hand 300 is only an example. The subject matter described herein can be applied or used on any part or parts of a body an animal that is a human or non-human.

In the example of FIG. 3B, a circuit 314 is configured to measure a resistance between first electrode 310 and second electrode 312. For example, circuit 314 may be a circuit used to measure a skin resistance between first electrode 310 and second electrode 312 when the electrodes are placed at different points on a body (e.g., on the skin). For example, first electrode 310 and second electrode 312 may be positioned on or to be in contact with different fingers of a hand, similar to the placement of first electrode 302 and second electrode 304 on hand 300 in the example of FIG. 3A. Even though circuit 314 is labeled "Signal Proc," it should be understood that circuit 314 may be any type of circuit, such as a conductance measurement circuit, a resistance measurement circuit, an ohmmeter, an electrical conductivity meter, a multimeter, and/or the like, etc.

In the example of FIG. 3B, circuit 314 is interfaced with interface module 316. Interface module 316 may be an apparatus similar as described with regards to FIG. 1, FIGS. 2A-2C, and/or the like. In some implementations, interface module 316 may be different from the example apparatuses described in FIG. 1, FIGS. 2A-2C and/or the like. Interface module 316 may include a different set or a subset of components or units shown in FIG. 1. For example, interface module 316 may include one or more of communication device 15, processor 11, memory 12, battery, communication connection (e.g., for communicating or interfacing with circuit 314, etc. Interface module 316 may include any combination of an antenna, a power source (e.g., battery or generator from heat and/or motion), a processing module (e.g., a system on chip), a communication interface (e.g., Bluetooth low energy, etc.), a crystal, and/or the like. Interface module 316 may receive information indicative of a resistance (e.g. a skin resistance) between first electrode 310 and second electrode 312 from circuit 314. In this manner, information indicative of a resistance, such as a skin resistance, between first electrode 310 and second electrode 312 may be communicated to one or more separate apparatuses, similar as described regarding FIGS. 2A-2C.

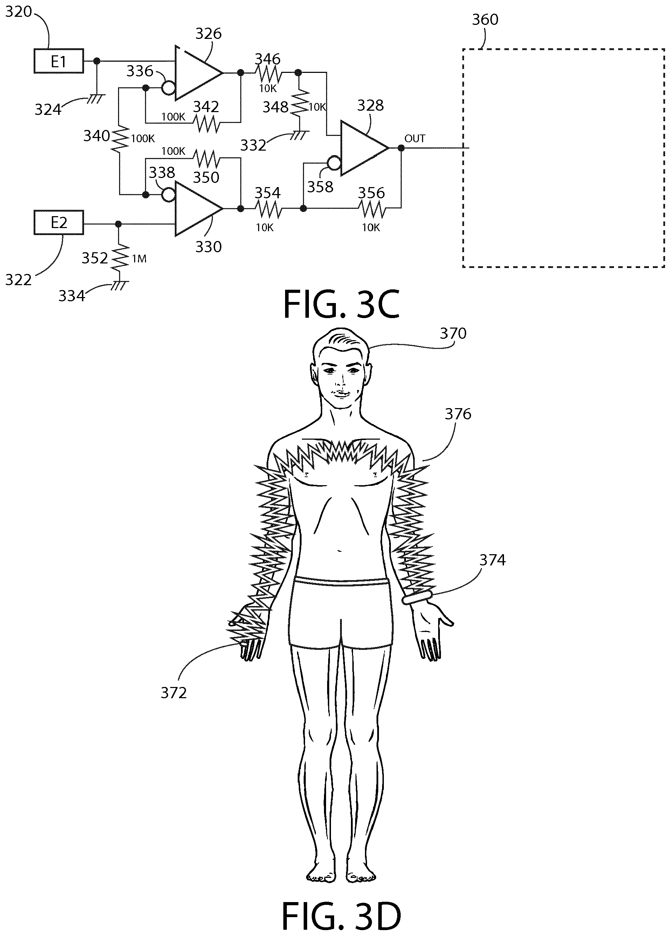

The example of FIG. 3C illustrates an example circuit (e.g., an example implementation of circuit 314). In the example of FIG. 3C, the example circuit is configured to measure a skin resistance between first electrode 320 and second electrode 322. For example, the example circuit may measure a skin resistance between first electrode 320 and second electrode 322 when the electrodes are placed at different points on skin. For example, first electrode 320 and second electrode 322 may be positioned on different fingers of a hand, similar to the placement of first electrode 302 and second electrode 304 on hand 300 in the example of FIG. 3A. It can be seen that the example circuit of FIG. 3C comprises operational amplifiers 326, 328, and 330; ground points 324, 332, and 334; resistors 340, 342, 346, 348, 350, 352, 354, and 356; and inverters 336, 338, and 358. Even though the example of FIG. 3C illustrates a particular example circuit, skin resistance may be measured with other configurations of circuits, different types of circuits (e.g., circuits designed specific for measuring skin resistance), generic circuits, and/or the like. In some implementations, software, such as firmware, may be executed on the circuits.

In the example of FIG. 3C, the example circuit is interfaced with interface module 360. Interface module 360 may be an apparatus similar as described with regards to interface module 316 of FIG. 3B. Interface module 360 may receive information indicative of a skin resistance between first electrode 320 and second electrode 322 from the example circuit of FIG. 3C. In this manner, information indicative of a resistance, such as a skin resistance, between first electrode 320 and second electrode 322 may be communicated to a separate apparatus, similar as described regarding FIGS. 2A-2C and FIG. 3B.

In some circumstances, an electrode may be configured to measure skin resistance from a different portion of skin than previously described. For example, a first electrode may be placed on a finger, while the second electrode is placed on a wrist, the first and second electrodes may be placed on opposite limbs, and/or the like. In this manner, a skin resistance may be measured along different portions of the body.

In the example of FIG. 3D, first electrode 372 is in contact with the skin of the right hand index finger of person 370, and second electrode 374 is in contact with the skin of the left wrist of person 370 such that a skin resistance of person 370 between first electrode 372 and second electrode 374 may be measured when electrical path 376 is formed (e.g., when electrodes 372 and 374 are parts of the same circuit, such as electrodes 320 and 322 of the circuit in FIG. 3C). For example, it should be understood that an electrical path 376 with a measurable skin resistance exists on and/or in the skin of person 370 between first electrode 372 and second electrode 374. Even though first electrode 372 and second electrode 374 are illustrated with a ring configuration and a bracelet configuration, it should be understood that first electrode 372 and second electrode 374 may be any configuration of electrode.

It is known that various factors can influence the resistance and/or the conductance of skin. For example, the amount of sweat on the skin, a person's diet, fitness level, etc. may cause a variation in resistance and/or conductance measurements. The variation of skin resistance and/or skin conductance over time may be referred to as a galvanic skin response. It is known that the galvanic skin response of a person may be affected by the person's mood, response to external stimulation, and/or the like. For example, the rate of perspiration of a person is influenced by the person's sympathetic nervous system. In this manner, the sympathetic nervous system may influence the galvanic skin response. In some circumstances, it may be desirable to determine the galvanic skin response of a person. For example, the determination of the galvanic skin response of a person may be used to determine the person's mood, the effect of external stimulation on a person, and/or the like. In at least one example embodiment, an apparatus determines the galvanic skin response of a person. In at least one example embodiment, the determination of the galvanic skin response is based, at least in part, on a variation of the skin resistance of the person over time.

For example, a circuit, such as the example circuit of FIG. 3C, may take periodic skin resistance measurements of a person over time. These periodic measurements may be used to determine a galvanic skin response of the person. In at least one example embodiment, the determination of the galvanic skin response is based, at least in part, on a difference between the skin resistance from a predetermined skin resistance value. For example, the predetermined skin resistance value may be a previously measured value, an average value, a typical value, a calibrated value, and/or the like.

In current times, users' of apparatuses are increasingly using their apparatuses to monitor aspects of their health. For example, a user may utilize an application on a mobile device, a wearable device, and or the like to track their caloric intake, to monitor distances walked by the user, record the user's vital signs, and/or the like. In circumstances such as these, it may be desirable for the apparatus to receive information indicative of a galvanic skin response of the user. In this manner, the apparatus may utilize the information to monitor the user's galvanic skin response, track the user's galvanic skin response, determine the user's galvanic skin response, and/or the like. For example, the apparatus may receive information indicative of a galvanic skin response by way of a circuit. The information indicative of a galvanic skin responses may be a skin resistance, a variation in skin resistance, information derived from a determined galvanic skin response, and/or the like. In at least one embodiment, the apparatus comprises a circuit. In at least one example embodiment, the apparatus receives information indicative of a galvanic skin response by way of a communication channel. The apparatus and the communication channel may be similar as described regarding FIG. 1, FIGS. 2A-2C, and/or the like.

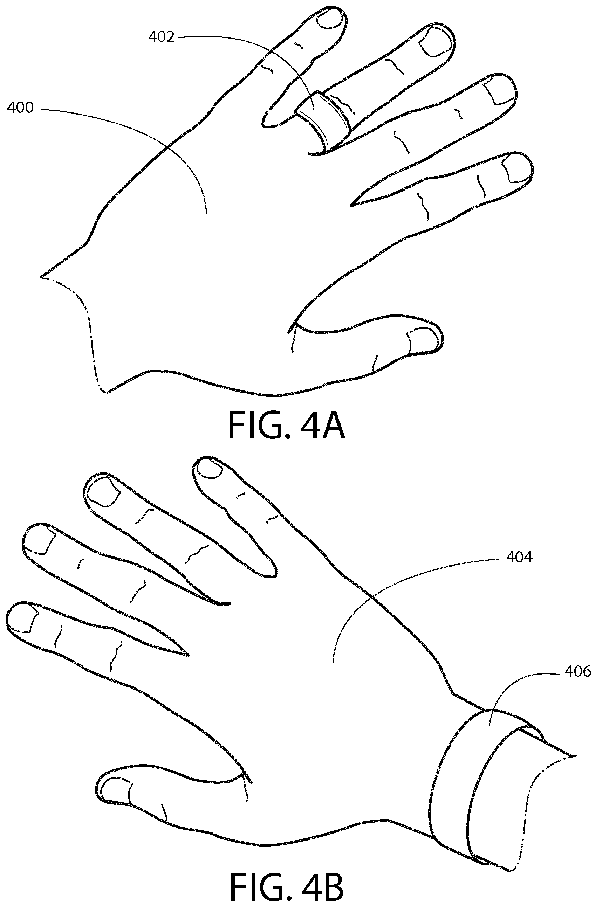

FIGS. 4A-4J are diagrams illustrating housings according to at least one example embodiment. The examples of FIGS. 4A-4J are merely examples. For example, the housing configurations may vary, the housing shapes may vary, the housing sizes may vary, electrode configuration and/or arrangement may vary, and/or the like. Any example housings in FIGS. 4A-4J may house electrodes 310 and 312, circuit 314, interface module 316, and/or other components, modules, or devices.

As previously described, an apparatus may comprise a housing. For example, an apparatus may comprise a housing configured to be in contact with or at least partially encircle a body part of a user of an apparatus. The example of FIG. 4A illustrates apparatus 402 worn by user of the apparatus 400. It can be seen that apparatus 402 comprises a housing configured to at least partially encircle the ring finger of user of the apparatus 400. Similarly, the example of FIG. 4B illustrates apparatus 406 worn by user of the apparatus 404. It can be seen that apparatus 406 comprises a housing configured to at least partially encircle the wrist of user of the apparatus 404.

As previously described, in some circumstances, a user of an apparatus may desire to determine the user's galvanic skin response, similar as described with regards to FIGS. 3A-3D. In circumstances such as these, it may be desirable for the housing of the apparatus to comprise electrodes for measuring the skin resistance of the user. For example, integrating the electrodes into the housing may reduce the bulk of the apparatus used to determine galvanic skin response, may allow for a self-contained apparatus, and/or the like. In at least one example embodiment, an apparatus comprises a housing, a first electrode, a second electrode, and at least one circuit configured to measure skin resistance between the first electrode and the second electrode.

Under typical circumstances, when measuring a skin resistance between two electrodes, the measurement is most accurate when the first electrode and the second electrode are electrically insulated from one another. As such, it may be desirable to configure a housing comprising a first electrode and a second electrode in a manner where the first electrode and the second electrode are separated by an electrically insulating material. As used herein, there term "electrically insulating material" includes any material, such as space, air, non-conductive or insulator material (e.g., foam, rubber, plastic, ceramic, etc.). "Electrically insulating material" may also include semi-conducting material, (i.e., in non-conducting states). For example, a housing may comprise an inner surface and an outer surface. A first electrode may be disposed upon the inner surface of the housing, and a second electrode may be disposed upon the outer surface of the housing. In such an example, the electrode disposed on the inner surface of the housing and the electrode disposed on the outer surface of the housing may be separated by an electrically insulating material. An electrically insulating material may refer to a material through which electric charges do no flow freely. Examples of electrical insulating materials include materials with a high resistivity, such as glass, paper, Teflon, many polymers, fiberglass, rubber, and/or the like. With respect to a housing configured to encircle a body part of a user of the apparatus, an inner surface may refer to a surface of the housing configured to at least partially contact the body part, and an outer surface may refer to a surface of the housing is configured to avoid contact with the body part. For example, if the housing of the apparatus is configured as a ring, the inner portion of the ring which contacts the finger may comprise an inner surface, and the remainder of the surface of the ring may comprise an outer surface.

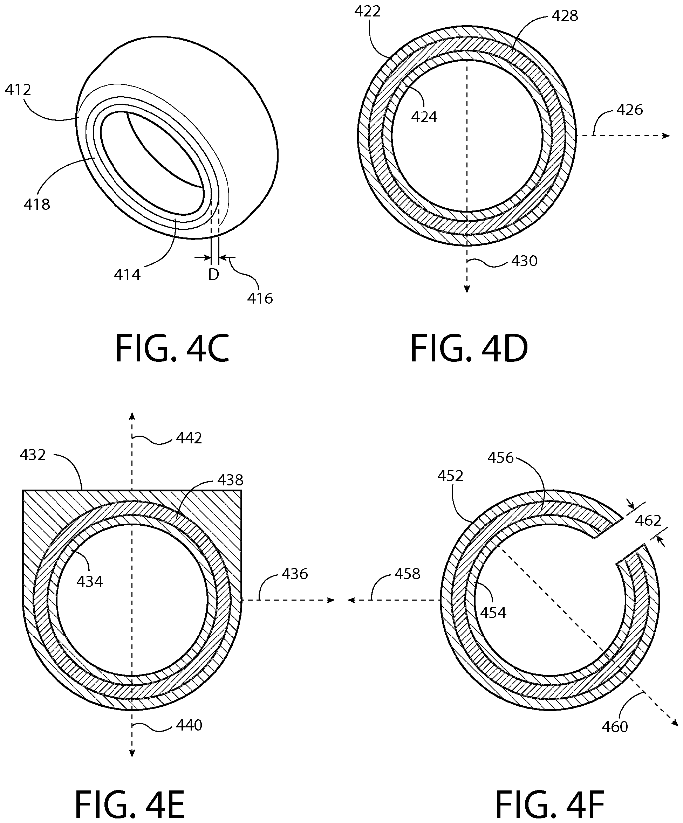

The example of FIG. 4C illustrates a perspective view of a housing configured to be in contact with or at least partially encircle a body part of a user of the apparatus. For example, the body part may be at least one of a finger, a wrist, an ankle, a neck, a waist, a head, a limb, toe, a torso, and/or the like. In the example of FIG. 4C, the housing comprises outer surface 412 and inner surface 414.

It should be understood that in the example of FIG. 4C, an electrode is disposed on at least part of outer surface 412. An electrode disposed on an outer surface of a housing may be referred to as an outer electrode. It should be understood that in the example of FIG. 4C, the outer electrode is disposed on at the at least part of outer surface 412 exclusive of disposition on the inner surface 414. In this manner, the outer electrode of the housing of FIG. 4C fails to be disposed on any part of inner surface 414. Each of the outer surface 412 and the inner surface 414 may be made of, coated or covered entirely or partially with one or more electrically conductive materials (conductive surface), may be made of, coated or covered entirely or partially with one or more electrically insulating materials (insulating surface), or may include any combination regions of conductive surface and insulating surface. A conductive surface may be use as an electrode in some implementations.

It should be understood that in the example of FIG. 4C, an electrode is disposed longitudinally on at least part of inner surface 414. In this manner, the electrode disposed on inner surface 414 is longitudinally contiguous. An electrode disposed on an inner surface of a housing may be referred to as an inner electrode. It should be understood that in the example of FIG. 4C, the inner electrode is disposed on at the at least part of inner surface 414 exclusive of disposition on the outer surface 412. In this manner, the inner electrode of the housing of FIG. 4C fails to be disposed on any part of outer surface 412.

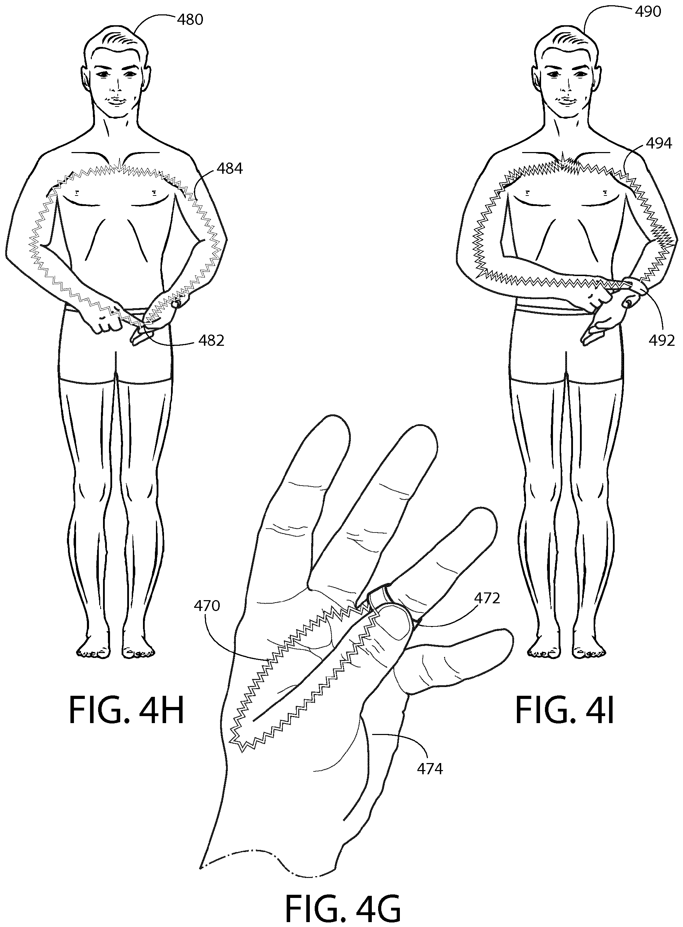

In the example of FIG. 4C, it can be seen that outer surface 412 and inner surface 414 are separated a distance 416 by material 418. It should be understood that in the example of FIG. 4C, material 418 is an electrically insulating material that separates the outer electrode disposed on outer surface 412 from the inner electrode disposed on inner surface 414. In this manner, a circuit (e.g., circuit 314) may measure a skin resistance between the inner electrode and the outer electrode when the housing of FIG. 4C is worn, similar as described regarding FIGS. 4G-4I. The skin resistance and/or other information may be communicated by an interface module (e.g., interface module 316) with another apparatus or device. The circuit and/or interface module may be housed or embedded anywhere in the housing of FIG. 4C (e.g., the circuit and/or interface module may be at least partially embedded in material 418).

In some circumstances, a user may wish for an apparatus to inform the user of a skin resistance measurement independently of communication with a separate apparatus. In circumstances such as these, an apparatus may communicate information indicative of a skin resistance measurement directly to the user. For example, the apparatus may cause display of a visual indicator on a display comprised by the apparatus, may cause rendering of an audible indicator by a sound generating device comprised by the apparatus, may cause rendering of a haptic indicator by way of a haptic feedback device comprised by the apparatus, and/or the like. Collectively, any of a display device, a sound generating device, a haptic generating device, or a light output device and the like may be referred to as an indication device.

In some circumstances, the information indicative of the skin resistance measurement may indicate a value of the skin resistance measurement. For example, a visual indicator may include a light output and/or a visual reference such as a chart, a numeral, and/or the like, a haptic indicator may vibrate with a particular rhythm, an audible indicator may speak with a particular value, and/or the like. In this manner, the apparatus may operate as a stand-alone apparatus. In other circumstances, the information indicative of the skin resistance measurement may simply indicate the measurement has been made. For example, a simple visual indicator may blink, an audio indicator may beep in a generic manner, and/or the like. In this manner, the user may be alerted of the measurement, and the user may determine a value of the measurement in another manner. For example, the user may activate a different mode on a stand-alone apparatus that provides more detail, the user may cause the apparatus to communicate the value to a separate apparatus, and/or the like. Examples of stand-alone apparatuses may be, for example, apparatus 200 (FIG. 2A), apparatus 210 (FIG. 2B), and the like that include the like of electrodes 310 and 312, circuit 314, and optional interface module 316 (FIG. 3B).

In the example of FIG. 4C, it can be seen that at least part of outer surface 412 is concentric with at least part of inner surface 414. In this manner, at least part of an outer electrode (e.g., electrode 310) disposed on outer surface 412 is concentric with at least part of an inner electrode (e.g., electrode 312) disposed on inner surface 414. In the example of FIG. 4C, it can be seen that material 418 is concentric with outer surface 412 and inner surface 414. In this manner, material 418 forms a concentric separation between the inner electrode and the outer electrode of FIG. 4C.

It should be understood that the components of a housing similar as described regarding FIG. 4C may be constructed of various material, with various dimensions, and/or the like depending on the particular application. For example, a housing configured as a ring may have an inner surface sized similar to an average ring, whereas a housing configured as a bracelet may have an inner surface sized to accommodate the size and shape of an average woman's wrist, an average man's wrist, and/or the like. In another example, a housing may be configured to a particular standard ring or bracelet size, may be configured with an adjustable size, and/or the like. Similarly, the spacing between the inner and the outer surfaces may be separated by a particular thickness and/or length of insulating material. For example, certain spacing between electrodes may achieve a particular electrical characteristic, such as decreased capacitance between the electrodes, less opportunity for shorting between the electrodes, and/or the like.

In some circumstances, when measuring a skin resistance, an electrode may benefit from contacting a larger portion of skin. In circumstances such as these, it may be desirable for a housing, such as a ring, to have a continuously toroidal shape. For example, an inner surface of the housing may make more contact with the body part. In this manner, an electrode disposed along the housing may make better electrical contact with the body part.

The example of FIG. 4D illustrates a section view of a housing having a continuously toroidal shape configured to completely encircle a body part of a user of the apparatus. For example, the housing of FIG. 4D may be sized as a ring, and the housing of FIG. 4D may completely encircle a finger. In the example of FIG. 4D, the housing (which may be similar to housing 202 of FIG. 2A, housing 212 of FIG. 2B, and/or the like) comprises outer surface 422 and inner surface 424. It should be understood that in the example of FIG. 4D, an electrode (e.g., electrode 310) is disposed on at least part of outer surface 422. It should be understood that in the example of FIG. 4D, an electrode (e.g., electrode 312) is disposed on at least part of inner surface 424. In the example of FIG. 4D, it can be seen that outer surface 422 and inner surface 424 are separated by material 428. It should be understood that in the example of FIG. 4D, material 428 is an electrically insulating material that separates the outer electrode disposed on outer surface 422 from the inner electrode disposed on inner surface 424. In this manner, a circuit (e.g., circuit 314) may measure a skin resistance between the inner electrode and the outer electrode when the housing of FIG. 4D is worn, similar as described regarding FIGS. 4G-4I. The skin resistance and/or other information may be communicated by an interface module (e.g., interface module 316) with another apparatus or device. The circuit and/or interface module may be housed or embedded anywhere in the housing of FIG. 4D (e.g., the circuit and/or interface module may be at least partially embedded in material 428).

Even though the housing of FIG. 4D is illustrated with an approximately perfectly toroidal shape, it should be understood that the housing of FIG. 4D may have an irregularly toroidal and/or partially toroidal shape, such as a toroidal polyhedron shape, an ellipsoid shape, and ovoid shape, and or the like. In the example of FIG. 4D, it can be seen that vector 426 lying normal to outer surface 422 fails to pass through inner surface 424, whereas vector 430 lying normal to inner surface 424 passes through outer surface 422. In this manner, outer surface 422 may be distinguished from inner surface 424.

As previously described, an apparatus may comprise a display. For example, an apparatus may have a configuration similar to apparatus 200 of FIG. 2A which includes a display similar to display 204, may have a configuration similar to apparatus 210 of FIG. 2B which includes a display similar to display 214, and/or the like. In this manner the apparatus may operate in a stand-alone mode similarly as previously described, the apparatus may cause display of a visual indicator on a display comprised by the apparatus, may cause rendering of an audible indicator by a sound generating device comprised by the apparatus, may cause rendering of a haptic indicator by way of a haptic feedback device comprised by the apparatus, and/or the like. In circumstances such as these, the display may be best viewed when the housing is worn with a particular orientation. For example, a display on a housing with a ring configuration may be best viewed with the housing is oriented with the display approximately parallel with the back of the hand. In circumstances such as these, it may be desirable for a housing, such as a ring, to have an outward protrusion from the outer surface of the housing. For example, an outward protrusion from the outer surface of the housing may be used to house components, such as display, serve as a tactile indicator of orientation, may comprise a display element, and/or the like.

The example of FIG. 4E illustrates a section view of a housing having an outward protrusion. In the example of FIG. 4E, the housing (which may be similar to housing 202 of FIG. 2A, housing 212 of FIG. 2B, and/or the like) comprises outer surface 432 and inner surface 434. It should be understood that in the example of FIG. 4E, an electrode (e.g., electrode 310) is disposed on at least part of outer surface 432. It should be understood that in the example of FIG. 4E, an electrode (e.g., electrode 312) is disposed on at least part of inner surface 434. In the example of FIG. 4E, it can be seen that outer surface 432 and inner surface 434 are separated by material 438. It should be understood that in the example of FIG. 4E, material 438 is an electrically insulating material that separates the outer electrode disposed on outer surface 432 from the inner electrode disposed on inner surface 434. In this manner, a circuit (e.g., circuit 314) may measure a skin resistance between the inner electrode and the outer electrode when the housing of FIG. 4E is worn, similar as described regarding FIGS. 4G-4I. The skin resistance and/or other information may be communicated by an interface module (e.g., interface module 316) with another apparatus or device. The circuit and/or interface module may be housed or embedded anywhere in the housing of FIG. 4E (e.g., the circuit and/or interface module may be at least partially embedded in material 438).

Even though the outward protrusion of FIG. 4E is illustrated with a particular shape, it should be understood that the outward protrusion of housing of FIG. 4E may have any shape. In the example of FIG. 4E, it can be seen that vector 436 lying normal to outer surface 432 fails to pass through inner surface 434, whereas vector 440 lying normal to inner surface 434 passes through outer surface 432. In this manner, outer surface 432 may be distinguished from inner surface 434. It can be seen that the outward protrusion of housing of FIG. 4E has flat shape perpendicular to vector 442. In this manner, the outward protrusion of the housing of FIG. 4E is distinct from the remainder of the outer surface 432 such that the outward protrusion may serve as a tactile indicator of the orientation of the housing.

In some circumstances, a user of an apparatus comprising housing may desire for the housing to be used on multiple body parts. For example, the user may wish to use a housing configured as a ring on different fingers of different sizes. In circumstances such as these, it may be desirable for a housing, such as a ring, to have a discontinuously toroidal shape. For example, a housing with a discontinuously toroidal shape may have more flex, fit adjustment, and/or the like to accommodate body parts of different sizes. A discontinuously toroidal shape may refer to a toroidal shape comprising a gap within the toroidal shape.

The example of FIG. 4F illustrates a section view of a housing having a discontinuously toroidal shape configured to partially encircle a body part of a user of the apparatus without completely encircling the body part. For example, the housing of FIG. 4F may be sized as a ring, and the housing of FIG. 4F may completely encircle a finger. In the example of FIG. 4F, the housing (which may be similar to housing 202 of FIG. 2A, housing 212 of FIG. 2B, and/or the like) comprises outer surface 452 and inner surface 454. It should be understood that in the example of FIG. 4F, an electrode (e.g., electrode 310) is disposed on at least part of outer surface 452. It should be understood that in the example of FIG. 4F, an electrode (e.g., electrode 310) is disposed on at least part of inner surface 454. In the example of FIG. 4F, it can be seen that outer surface 452 and inner surface 454 are separated by material 456. It should be understood that in the example of FIG. 4F, material 456 is an electrically insulating material that separates the outer electrode disposed on outer surface 452 from the inner electrode disposed on inner surface 454. In this manner, a circuit (e.g., circuit 314) may measure a skin resistance between the inner electrode and the outer electrode when the housing of FIG. 4F is worn, similar as described regarding FIGS. 4G-4I. The skin resistance and/or other information may be communicated by an interface module (e.g., interface module 316) with another apparatus or device. The circuit and/or interface module may be housed or embedded anywhere in the housing of FIG. 4F (e.g., the circuit and/or interface module may be at least partially embedded in material 456).

Even though the housing of FIG. 4F is illustrated with an approximately perfectly toroidal shape, it should be understood that the housing of FIG. 4F may have an irregularly toroidal shape, such as a toroidal polyhedron shape, an ellipsoid shape, an ovoid shape, and or the like. In the example of FIG. 4F, it can be seen that vector 458 lying normal to outer surface 452 fails to pass through inner surface 454, whereas vector 460 lying normal to inner surface 454 passes through outer surface 452. In this manner, outer surface 452 may be distinguished from inner surface 454. It can be seen that the housing of FIG. 4F has gap 462. In this manner, the housing of FIG. 4F has a shape that is not of a ring.

The example of FIG. 4G illustrates user 474 using housing 472 comprising an inner electrode and an outer electrode similar as described regarding FIGS. 4A-4F. In the example of FIG. 4G, the inner electrode of housing 472 is in contact with the skin of the left hand ring finger of user 474, and the outer electrode of housing 472 is in contact with the skin of the thumb of user 474 such that a skin resistance of user 474 between the inner electrode and the outer electrode may be measured. For example, it should be understood that when both inner and outer electrodes (e.g., both points) are in contact with user 474, an electrical path 470 is formed such that a measurable skin resistance exists on and/or in the skin of user 474 between both points, i.e., the inner electrode and the outer electrode of housing 472. Electrical path 470 is likely to be the shortest electrical path through user 474 between the inner electrode and the outer electrode.

The example of FIG. 4H illustrates user 480 using housing 482 comprising an inner electrode and an outer electrode similar as described regarding FIGS. 4A-4F. In the example of FIG. 4H, the inner electrode of housing 482 is in contact with the skin of the left hand ring finger of user 480, and the outer electrode of housing 482 is in contact with the skin of the right hand index finger of user 480 such that a skin resistance of user 480 between the inner electrode and the outer electrode may be measured. For example, it should be understood that an electrical path 484 with a measurable skin resistance exists on and/or in the skin of user 480 between the inner electrode and the outer electrode of housing 482.

The example of FIG. 4I illustrates user 490 using housing 492 comprising an inner electrode and an outer electrode similar as described regarding FIGS. 4A-4F. In the example of FIG. 4I, the inner electrode of housing 492 is in contact with the skin of the left hand wrist of user 490, and the outer electrode of housing 492 is in contact with the skin of the right hand index finger of user 490 such that a skin resistance of user 490 between the inner electrode and the outer electrode may be measured. For example, it should be understood that an electrical path 494 with a measurable skin resistance exists on and/or in the skin of user 494 between the inner electrode and the outer electrode of housing 492.

The subject matter herein is not limited to ring type apparatuses and the like. Apparatuses of any shape, in ring form, semi-ring form, and non-ring form may be implemented. For example, in some circumstances, it may be desirable for a housing to have a shape in which electrodes comprised by the housing fail to encircle a part of a user of the apparatus. For example, a user may desire to use an apparatus without wearing it, or may desire to wear an apparatus, such as a pendant shaped apparatus, on a chain, a necklace, a string, a lanyard, and/or the like.

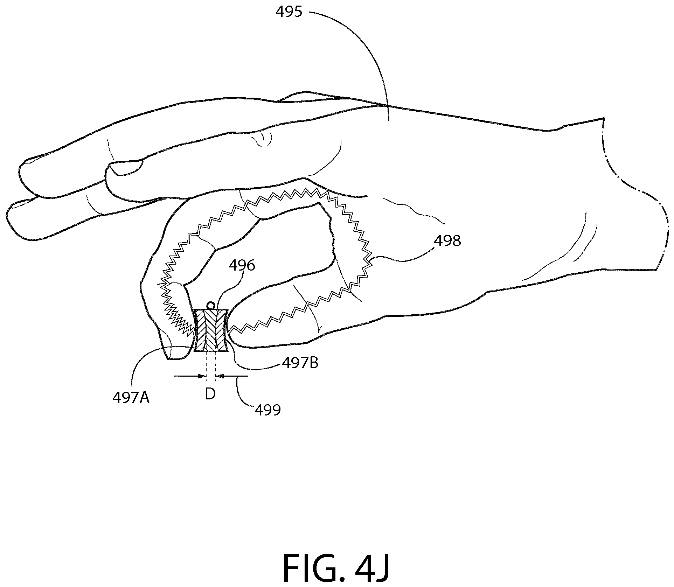

The example of FIG. 4J illustrates a section view of a housing having, for example, a pendant shape being held between two parts of a user, such as a thumb and a finger of user 495. In the example of FIG. 4J, the housing comprises first surface 497A and second surface 497B. It should be understood that in the example of FIG. 4J, a first electrode is disposed on at least part of first surface 497A. It should be understood that in the example of FIG. 4J, a second electrode is disposed on at least part of second surface 497B. To illustrate that the description herein for ring type apparatuses is applicable to non-ring type apparatuses, and vice versa, one of the surfaces 497A and 497B may be referred to as an inner surface, and the other surface may be referred to as an outer surface. Similarly, one of the first and second electrodes may be referred to as an inner electrode, and the other electrode may be referred to as an outer electrode.

In the example of FIG. 4J, the first electrode of the housing is shown in contact with the skin of, for example, an index finger of user 495, and the second electrode of the housing is shown in contact with the skin of a thumb of user 495 such that a skin resistance (e.g., through path 498) of user 495 between the first electrode and the second electrode may be measured. It should be understood that an electrical path (e.g., path 498) with a measurable skin resistance exists between the first electrode and the second electrode of the housing. In the example of FIG. 4J, it can be seen that first surface 497A and second surface 497B are separated by material 496. It should be understood that in the example of FIG. 4J, material 496 is an electrically insulating material that separates the first electrode disposed on first surface 497A from the second electrode disposed on second surface 497B. In this manner, a circuit (e.g., circuit 314) may measure a skin resistance between the first electrode and the second electrode when the housing of FIG. 4J is in contact with two points of a user, similar as illustrated FIG. 4J. The skin resistance and/or other information may be communicated by an interface module (e.g., interface module 316) with another apparatus or device. The circuit and/or interface module may be housed or embedded anywhere in the housing of FIG. 4J (e.g., the circuit and/or interface module may be at least partially embedded in material 496).

In some implementations, material 496 covers surfaces 497A and 497B on all sides except the opposing sides of surfaces 497A and 497B, even though this is not visible in the section view of FIG. 4J. In this manner, a user may be unlikely to inadvertently create an electrical path between first surface 497A and second surface 497B unintentionally (for example, by laying a finger across the top of the pendant shaped apparatus). Even though the housing of FIG. 4J is illustrated with an approximately symmetrical shape, it should be understood that the housing of FIG. 4J may have an irregular and/or nonsymmetrical shape. Even though FIG. 4J illustrates an apparatus being held in a particular manner, the apparatus of FIG. 4J may be held or positioned in other manners in which a skin resistance measurement may be made for a user. For example, the user may hold the apparatus of FIG. 4J such that a finger on the left hand is in contact with the first electrode, and another part of the user, such as the user's face, arm, a finger on the right hand, etc. is in contact with the second electrode.

It can be seen that in the example of FIG. 4J that material 496 separates first surface 497A and second surface 497B by a distance 499. In this manner, an electrical resistance measured between the first electrode and the second electrode must have an electrical path that is longer than distance 499. For example, it can be seen that electrical path 498 is longer than distance 499 in the example of FIG. 4J. In this manner, the electrical path 498 longer than distance 499 may allow for a more consistent skin resistance measurement, a more accurate skin resistance measurement, and/or the like, than an electrical path that corresponds with distance 499.

FIG. 4H-4J show that when any two parts of a user are in contact directly and/or indirectly with the electrodes on the apparatus (e.g., the inner and outer electrodes on the inner and outer surfaces, respectively), the shortest electrical path (e.g., path 306, 470, 484, 494, 498 or another path) formed with the electrodes through the user is longer than any path through the apparatus.

Even though FIGS. 4A-4J show specific points of contact and specific electrical paths from which skin resistance may be measured, it should be understood that the examples of FIGS. 4A-4J are merely examples, and that other skin paths and/or points of contact may be used for measuring skin resistance.

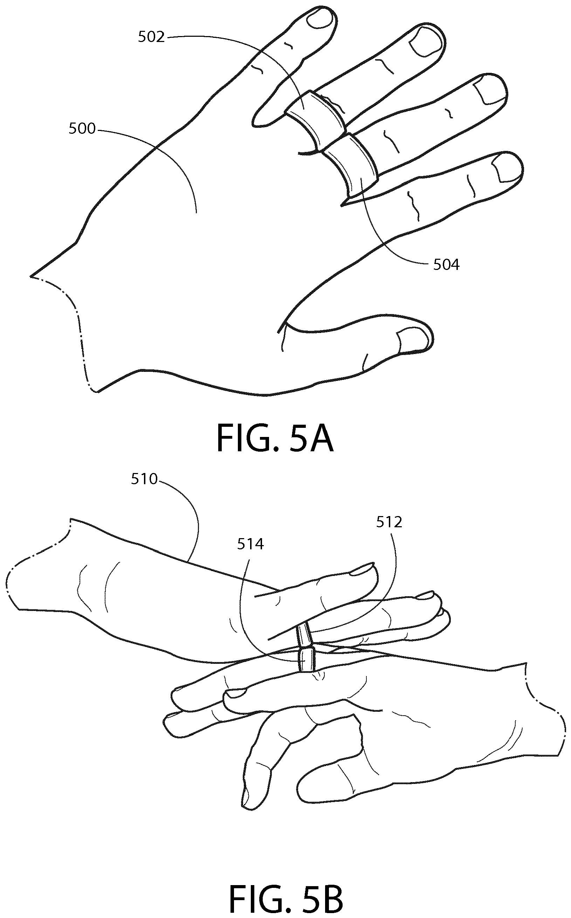

FIGS. 5A-5E are diagrams illustrating separate housings according to at least one example embodiment. The examples of FIGS. 5A-5E are merely examples. For example, the housing configurations may vary, the housing shapes may vary, the housing sizes may vary, and/or the like.

As previously described, in some circumstances a skin resistance may be measured by way of a housing comprising an inner electrode and an outer electrode. In circumstances such as described with regards to FIG. 4A-4I, only the inner electrode is able to completely encircle a part of the user's body. In some circumstances, it may be desirable for the outer electrode to make more contact with the user's body than typically capable in the examples of FIGS. 4A-4I. For example, more contact with the user's body may allow for a more consistent skin resistance measurement, a more accurate skin resistance measurement, and/or the like. In circumstances such as these, more contact may be achieved by way of a separate housing configured to at least partially encircle a different body part of the user.

For example, the separate housing may comprise an inner surface configured to at least partially contact the different body part and an outer surface of the separate housing is configured to avoid contact with the body part. In such an example, the inner surface and the outer surface may comprise an electrically conductive material, and an electrical conductor that extends between at least part of the electrically conductive material of the inner surface of the separate housing and at least part of the electrically conductive material of the outer surface of the separate housing. In this manner the outer surface and the inner surface may comprise an electrical path to the skin of the user when the separate housing is worn by the user. In another example, a separate housing may consist entirely of electrically conductive material. In this manner, the separate housing may comprise an electrical path to the skin of the user when the separate housing is worn by the user.

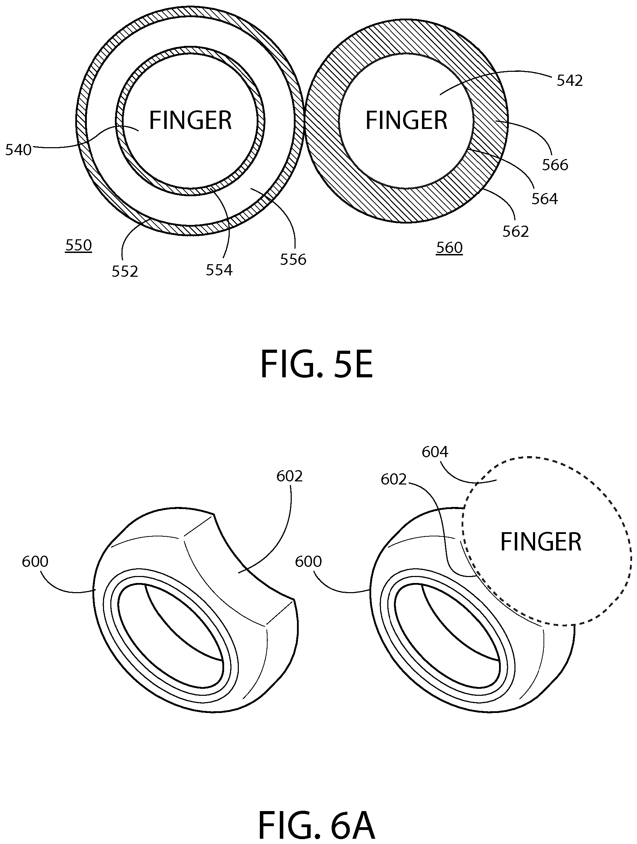

The example of FIG. 5A illustrates user 500 using housing 502 on the user's ring finger and separate housing 504 on the user's middle finger. In the example of FIG. 5A, housing 502 and separate housing 504 are similar as described regarding FIG. 5E. In the example of FIG. 5A, it should be understood that housing 502 and separate housing 504 are making electrical contact across their outer surfaces, similar as described regarding FIG. 5E. In this manner, a circuit may measure a skin resistance of an electrical path (not shown) between the user's ring finger and middle finger from the inner surface of housing 502 and the inner surface of separate housing 504.

The example of FIG. 5B illustrates user 510 using housing 512 on the user's right hand ring finger and separate housing 514 on the user's left hand ring finger. In the example of FIG. 5B, housing 512 and separate housing 514 are similar as described regarding FIG. 5E. In the example of FIG. 5B, it should be understood that housing 512 and separate housing 514 are making electrical contact across their outer surfaces, similar as described regarding FIG. 5E. In this manner, a circuit may measure a skin resistance of an electrical path (not shown) between the user's left hand ring finger and right hand ring finger from the inner surface of housing 512 and the inner surface of separate housing 514.

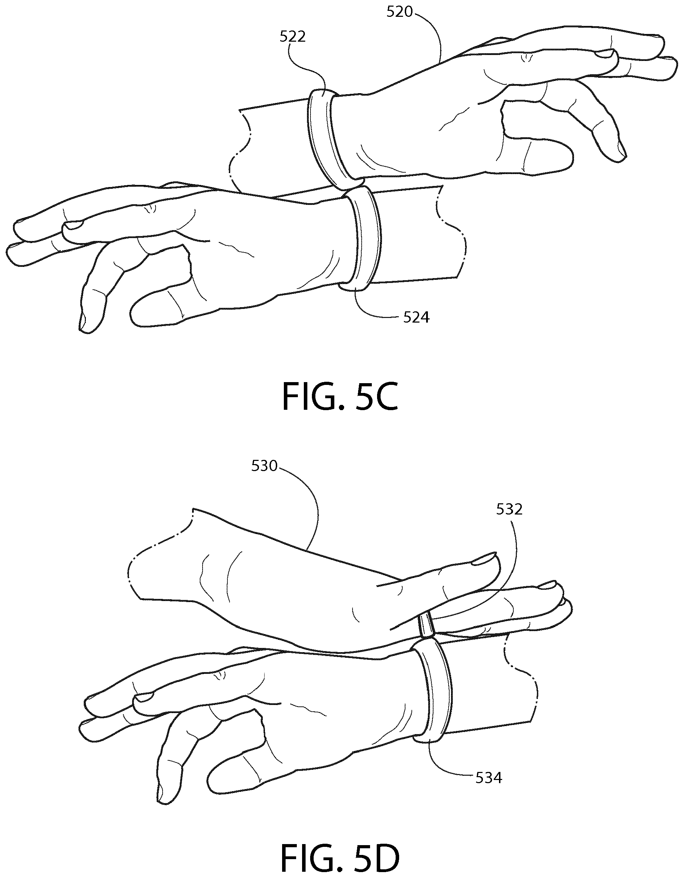

The example of FIG. 5C illustrates user 520 using housing 522 on the user's right wrist and separate housing 524 on the user's left wrist. In the example of FIG. 5C, housing 522 and separate housing 524 are similar as described regarding FIG. 5E. In the example of FIG. 5C, it should be understood that housing 522 and separate housing 524 are making electrical contact across their outer surfaces, similar as described regarding FIG. 5E. In this manner, a circuit may measure a skin resistance of an electrical path (not shown) between the user's left wrist and right wrist from the inner surface of housing 522 and the inner surface of separate housing 524.

The example of FIG. 5D illustrates user 530 using housing 532 on the user's right hand ring finger and separate housing 534 on the user's left wrist. In the example of FIG. 5D, housing 532 and separate housing 534 are similar as described regarding FIG. 5E. In the example of FIG. 5D, it should be understood that housing 532 and separate housing 534 are making electrical contact across their outer surfaces, similar as described regarding FIG. 5E. In this manner, a circuit may measure a skin resistance of an electrical path (not shown) between the user's left wrist and right hand ring finger from the inner surface of housing 532 and the inner surface of separate housing 534.

The example of FIG. 5E illustrates a section view of housing 550 and separate housing 560. In the example of FIG. 5E, housing 550 is configured to at least partially encircle and at least partially contact body part 540 of a user of the housing 550, and separate housing 560 is configured to at least partially encircle and at least partially contact body part 542 of the user of housing 560. Even though the example of FIG. 5E illustrates body parts 540 and 542 as fingers, it should be understood that body parts 540 and 542 may be any body parts, such as wrists, toes, limbs, ankles, and/or the like. Even though housing 550 and separate housing 560 are illustrated as perfectly toroidal, it should be understood that housing 550 and separate housing 560 may be other shapes, such as shapes described regarding FIGS. 4A-4J, shapes described regarding FIGS. 6A-6E, and/or the like.

In the example of FIG. 5E, housing 550 comprises outer surface 552 and inner surface 554. It should be understood that in the example of FIG. 5E, an electrode is disposed on at least part of outer surface 552. It should be understood that in the example of FIG. 5E, an electrode is disposed on at least part of inner surface 554. In the example of FIG. 5E, it can be seen that outer surface 552 and inner surface 554 are separated by material 556. It should be understood that in the example of FIG. 5E, material 556 is an electrically insulating material that separates the outer electrode disposed on outer surface 552 from the inner electrode disposed on inner surface 554. It should be understood that in the example of FIG. 5E that housing 550 is similar to housings described regarding FIGS. 4A-4J.

In the example of FIG. 5E, separate housing 560 comprises outer surface 562 and inner surface 564. It should be understood that in the example of FIG. 5E, an outer surface 562 and inners surface 564 comprise an electrically conductive material. In the example of FIG. 5E, it can be seen that material 566 extends between outer surface 562 and inner surface 564. It should be understood that in the example of FIG. 5E, material 566 is an electrically conductive material that extends from at least part of the electrically conductive material of inner surface 564 to at least part of the electrically conductive material of outer surface 562. In this manner, electrical current may flow between the electrically conductive material of inner surface 564 to the electrically conductive material of outer surface 562. In the example of FIG. 5E inner surface 564 is configured to at least partially contact body part 542 and outer surface 562 is configured to avoid contact with body part 542.

In the example of FIG. 5E, it can be seen that outer surface 552 of housing 550 is in contact with outer surface 562 of housing 560. In this manner, electrical current may flow between the electrode disposed on outer surface 562 and housing 560. As housing 560 may serve as an extension of the outer electrode of housing 550. In this manner, a circuit may measure a skin resistance between the inner electrode of housing 550 and inner surface 564 of separate housing 560 in circumstances where housing 550 and separate housing 560 are worn, similar as described regarding FIGS. 5A-5D.

In the example of FIG. 5E and other examples, note that the user may control when to allow contacting either electrode or both electrodes. For example, the user may control when to contact an inner electrode by controlling when to wear housing 550, which causes the user to contact the inner surface 554 and an electrode disposed thereon. The user may control when to contact an external electrode by controlling when to touch the outer surface 552, with or without a separate housing 560.

FIGS. 6A-6E are diagrams illustrating housings according to at least one example embodiment. The examples of FIGS. 6A-6E are merely examples. For example, the housing configurations may vary, the housing shapes may vary, the housing sizes may vary, and/or the like.

As previously described, in some circumstances, it may be desirable for an electrode to contact a larger portion of skin. In some circumstances, a housing may be shaped to allow contact with a larger portion of skin. For example, the outer surface of a housing may comprise a channel configured to partially encircle a body part of a user. A channel may refer to a groove, and indention, and/or the like. For example, a housing similar as described regarding FIGS. 4A-4J may comprise a channel shaped in correspondence with a finger. In a least one example embodiment, a housing is a ring configured to at least partially encircle a different finger of the user and the channel is sized substantially the same as the inner surface of the housing.

The example of FIG. 6A illustrates housing 600. In the example of FIG. 6A, housing 600 is similar to housings described regarding FIGS. 4A-4J. In the example of FIG. 6A, the outer surface of housing 600 comprises channel 602. In the example of FIG. 6A, channel 602 is shaped to correspond with body part 604. Even though body part 604 is illustrated as a finger, body part 604 may be any body part, such as a wrist, and ankle, and/or the like. Channel 602 and/or the like may be implemented to, for example, provide an increased contact area (i.e., an increased surface area) that contacts the skin of a user's body part (e.g., finger 604).

In some circumstances, it may be possible for an electrode, a portion of a housing, and/or the like to contact multiple portions of skin, other objects, and/or the like. For instance, an electrode may contact skin on multiple fingers, the webbing between the fingers, other body parts, jewelry, and/or the like. Such contact may be undesirable. For example, the extra skin contact may alter a particular electrical path of the skin between a first electrode and a second electrode. Such change in electrical path may lead to an inaccurate skin resistance measurement, an inconsistent skin resistance measurement, and/or the like. In circumstances such as these, it may be desirable to electrically insulate at least a portion of an electrode, a housing, and/or the like such as to limit the points of contact of the electrode, the housing, and/or the like. In this manner, the point of contact of the electrode may avoid contact with undesired parts of the skin, other body parts, other objects such as jewelry, and/or the like. The portion of the electrode, the housing, and/or the like insulated may vary based on the shape, size, position, contact point, and/or the like of the electrode.

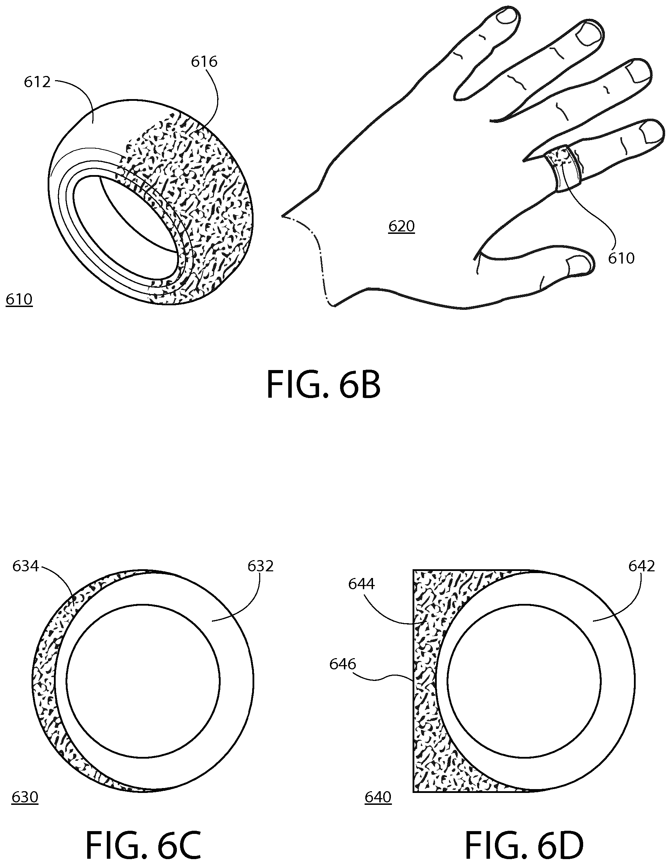

The example of FIG. 6B illustrates a housing 610. In the example of FIG. 6B, housing 610 is similar to housings described regarding FIGS. 4A-4J. In the example of FIG. 6C, outer surface 612 is covered by radial gap 616 that is electrically insulated from the outer electrode disposed upon outer surface 612 and the inner electrode of housing 610. A radial gap may refer to a portion of insulating material that covers a radially shaped electrode. In the example of FIG. 6B, housing 610 is shown being worn on the index finger of user 620 such that the radial gap faces user 620's middle finger. In this manner, if the middle finger of user 620 contacts the radial gap, an electrical current may fail to pass between the electrode disposed upon outer surface 612 and the middle finger of user 620.

The example of FIG. 6C illustrates separate housing 630. In the example of FIG. 6C, separate housing 630 is similar to housings described regarding FIGS. 5A-5C. In the example of FIG. 6C, the outer surface of the separate housing 630 comprises an electrically conductive surface 632 and an electrically insulated surface 634. In the example of FIG. 6C, electrically conductive surface 632 is a portion of the outer surface separate housing 630 that comprises an electrically conductive material. In the example of FIG. 6C, electrically insulating surface 634 is a portion of the outer surface of separate housing 630 that comprises an electrically insulating material. In this manner, a portion of skin contacting electrically insulating surface 634 may fail to carry a current between the portion of skin and electrically conductive surface 632.

As previously described, an electrode disposed on a housing may work best in a particular orientation. For example, the electrode may work best in a position where a radial gap is faced toward a portion of skin with which contact is undesirable. In circumstances such as these, it may be desirable for a tactile indicator to be disposed within the radial gap. In this manner, a user may readily identify the radial gap, orient the housing, and or the like, based on the tactile indicator. In at least one example embodiment, a housing comprises a tactile indicator disposed within the radial gap that tactilely differentiates the radial gap from the outer electrode. The tactile indicator may be a change of shape such as a protrusion, a change in material, a change in texture, and/or the like that may be felt as being distinct from the electrode. In at least one example embodiment, the electrically insulating surface of a separate housing comprises a tactile indicator that tactilely differentiates the electrically insulating surface of the separate housing from the electrically conductive surface of the separate housing.

The example of FIG. 6D illustrates separate housing 640. In the example of FIG. 6C, separate housing 640 is similar to housings described regarding FIGS. 5A-5C and FIG. 6C. In the example of FIG. 6D, the outer surface of the separate housing 640 comprises an electrically conductive surface 642 and an electrically insulated surface 644. In the example of FIG. 6D, electrically conductive surface 642 is a portion of the outer surface separate housing 640 that comprises an electrically conductive material. In the example of FIG. 6D, electrically insulating surface 644 is a portion of the outer surface of separate housing 640 that comprises an electrically insulating material. In this manner, a portion of skin contacting electrically insulating surface 644 may fail to carry a current between the portion of skin and electrically conductive surface 642. In the example of FIG. 6D. It can be seen that electrically insulating surface 644 includes an outward protrusion 646. In this manner, outward protrusion 646 may serve as a tactile indicator of the position of insulating surface 644 on separate housing 640.

As previously described, in some circumstances, it may be desirable for a housing to make contact with a separate housing. In some circumstances, it may be desirable for the housing to contact a larger portion of the separate housing, to contact a specific portion of the separate housing, and/or the like. In some circumstances, a housing may be shaped to allow contact with a larger portion of a separate housing. For example, the outer surface of a housing may comprise a channel configured to partially encircle the separate housing. For example, a housing similar as described regarding FIGS. 4A-4J and/or a separate housing similar as described regarding FIGS. 5A-5C, may comprise a channel shaped in correspondence with a housing and/or a separate housing. In at least one example embodiment, the outer surface of a housing comprises at least one channel that is configured to partially encircle a separate housing. In at least one example embodiment the channel is sized substantially the same as the outer surface of the separate housing. In at least one example embodiment, the outer surface of a separate housing comprises at least one channel that is configured to partially encircle a housing. In at least one example embodiment, the channel is sized substantially the same as the outer surface of the housing.

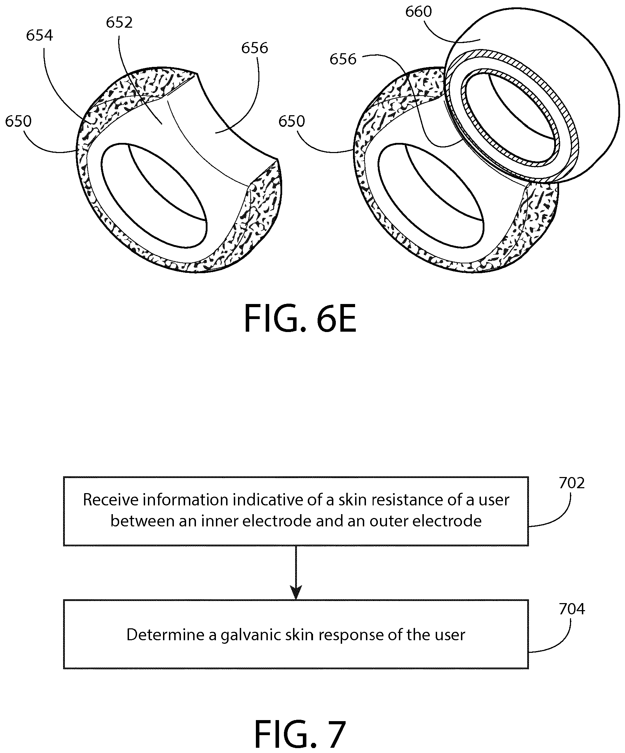

The example of FIG. 6E illustrates separate housing 650. In the example of FIG. 6E, housing 650 is similar to housings described regarding FIGS. 5A-5C. In the example of FIG. 6E, the outer surface of separate housing 650 comprises an electrically conductive surface 652 and an electrically insulated surface 654. In this manner, a portion of skin, a housing, and/or the like contacting electrically insulating surface 654 may fail to carry a current between the portion of skin, the housing, and/or the like, and electrically conductive surface 652. In the example of FIG. 6E, electrically conductive surface 652 of housing 650 comprises channel 656. In the example of FIG. 6E, channel 656 is shaped to correspond with housing 660. In the example of FIG. 6E, it should be understood that housing 660 is similar to housing described regarding FIGS. 4A-4J. In this manner, an electrical current may flow between electrically conductive surface 652 and the outer surface of housing 660.

FIG. 7 is a flow diagram illustrating activities associated with determining a galvanic skin response according to at least one example embodiment. In at least one example embodiment, there is a set of operations that corresponds with the activities of FIG. 7. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 7.

As previously described, it may be desirable to determine a galvanic skin response of a user of an apparatus.

At block 702, the apparatus receives, from a circuit, information indicative of a skin resistance between an inner electrode and an outer electrode. The receipt, the circuit, the skin resistance, the inner electrode, and the outer electrode may be similar as described regarding FIG. 1, FIGS. 2A-2C, FIGS. 3A-3D, FIGS. 4A-4J, FIGS. 5A-5E, and FIGS. 6A-6E.

At block 704, the apparatus determines a galvanic skin response of the user based, at least in part, on the skin resistance. The determination and the galvanic skin response may be similar as described regarding FIGS. 3A-3D, FIGS. 4A-4J, FIGS. 5A-5E, and FIGS. 6A-6E.

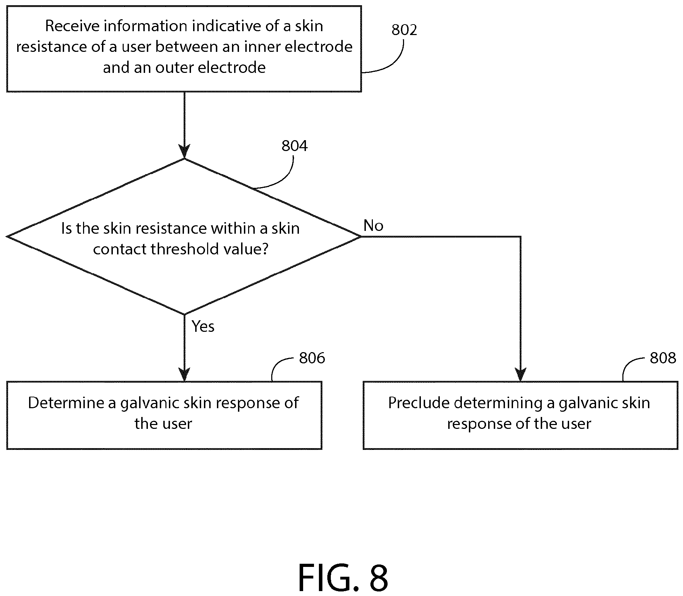

FIG. 8 is a flow diagram illustrating activities associated with determining a skin contact threshold value according to at least one example embodiment. In at least one example embodiment, there is a set of operations that corresponds with the activities of FIG. 8. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 8.

In some circumstances, an electrode may lack good contact with the surface of the skin, may lack any contact with the surface of the skin, may be electrically shorted to another electrode, and/or the like. During such circumstances, determination of a galvanic skin response may be inaccurate, may be inconsistent, may be impossible, and/or the like. In circumstances such as these, it may be desirable to determine that the skin resistance is within a skin resistance contact threshold and/or the skin resistance has not changed to be beyond a skin contact threshold value before determination of a galvanic skin response. A skin resistance contact threshold value may refer to a skin resistance measurement consistent with a minimum portion of skin in contact with an electrode, a maximum portion of skin in contact within an electrode, an electrode not being shorted to another electrode, an electrode not contacting an unintended portion of skin, and/or the like. For example, if the electrodes are shorted, the measured skin resistance may be low, and the skin resistance may be beyond a skin contact threshold value.

At block 802, the apparatus receives, from a circuit, information indicative of a skin resistance between an inner electrode and an outer electrode, similarly as described regarding block 702 of FIG. 7.

At block 804, the apparatus determines if the skin resistance is within a skin contact threshold value. If the apparatus determines the skin resistance is within a skin contact threshold value, flow proceeds to block 806. If the apparatus determines the skin resistance is beyond a skin contact threshold value, flow proceeds to block 808.

At block 806, the apparatus determines a galvanic skin response of the user based, at least in part, on the skin resistance, similarly as described regarding block 704 of FIG. 7. At block 808, the apparatus precludes determination of a galvanic skin response of the user.

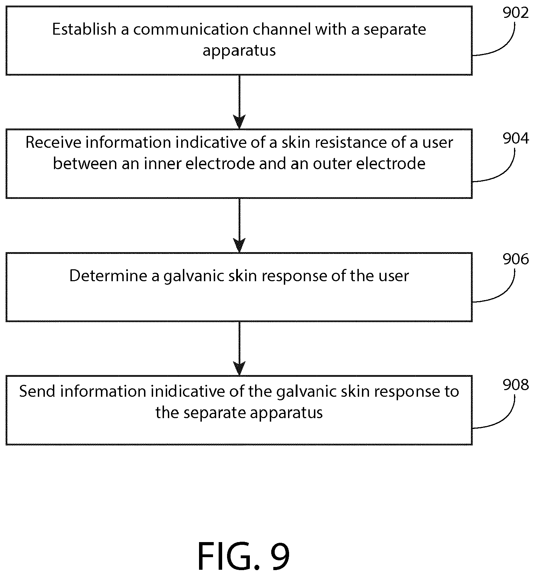

FIG. 9 is a flow diagram illustrating activities associated with sending information to a separate apparatus according to at least one example embodiment. In at least one example embodiment, there is a set of operations that corresponds with the activities of FIG. 9. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 9.

As previously described, in some circumstances an apparatus may send information indicative of a galvanic skin response to a separate apparatus.

At block 902, the apparatus establishes a communication channel with a separate apparatus. The establishment, the communication channel, and the separate apparatus may be similar as described regarding FIG. 1, FIGS. 2A-2C, FIGS. 3A-3D, FIGS. 4A-4J, FIGS. 5A-5E, and FIGS. 6A-6E.

At block 904, the apparatus receives, from a circuit, information indicative of a skin resistance between an inner electrode and an outer electrode, similarly as described regarding block 702 of FIG. 7. At block 906, the apparatus determines a galvanic skin response of the user based, at least in part, on the skin resistance, similarly as described regarding block 704 of FIG. 7.

At block 908, the apparatus sends information indicative of the galvanic skin response to the separate apparatus. The sending may be similar as described regarding FIGS. 2A-2C.

One or more example embodiments may be implemented in software, hardware, application logic or a combination of software, hardware, and application logic. The software, application logic, and/or hardware may reside on the apparatus, a separate device, or a plurality of separate devices. If desired, part of the software, application logic, and/or hardware may reside on the apparatus, part of the software, application logic and/or hardware may reside on a separate device, and part of the software, application logic, and/or hardware may reside on a plurality of separate devices. In an example embodiment, the application logic, software or an instruction set is maintained on any one of various computer-readable media.

If desired, the different functions discussed herein may be performed in a different order and/or concurrently with each other. For example, block 902 of FIG. 9 may be performed after block 900 of FIG. 9. Furthermore, if desired, one or more of the above-described functions may be optional or may be combined. For example, block 902 of FIG. 9 may be optional and/or combined with block 906 of FIG. 9.

Although various aspects of the present subject matter are set out in the independent claims, other aspects of the present subject matter comprise other combinations of features from the described example embodiments and/or the dependent claims with the features of the independent claims, and not solely the combinations explicitly set out in the claims.

It is also noted herein that while the above describes example embodiments, these descriptions should not be viewed in a limiting sense. Rather, there are variations and modifications which may be made without departing from the scope of the present disclosure. The scope of protection is defined by the appended claims.

## Claims

1. An apparatus (10, 200, 210, 220, 402, 406), comprising:
a wearable housing (202, 212, 472, 482, 492, 502, 512, 522, 532, 550, 600, 610) that comprises an inner surface (414, ,424, 434, 454, 554, 564) and an outer surface (412, , 422, 432, 452, 552, 562), the wearable housing configured to at least partially encircle a body part of a user, wherein the inner surface is configured to at least partially contact the body part;
an inner electrode (312) disposed on at least part of the inner surface;
an outer electrode (310) disposed on at least part of the outer surface such that the outer electrode is electrically insulated from the inner electrode wherein the inner and outer electrodes are configured to allow for skin resistance measurement; and
at least one circuit (314);
**characterized in that**:
the circuit (314) is configured to measure the skin resistance of a user between the inner electrode and the outer electrode.

2. The apparatus (10, 200, 210, 220, 402, 406) of claim 1, wherein the inner electrode and the outer electrode are configured such that an electrical skin resistance measured between the inner electrode and the outer electrode is measured over an electrical path (306, 376, 470, 484, 494, 498) that is longer than a distance between the inner electrode and the outer electrode through the apparatus.

3. The apparatus (10, 200, 210, 220, 402, 406) of any of claims 1-2, further comprising a communication device (15), wherein the communication device is configured to send information indicative of the skin resistance to a separate apparatus.

4. The apparatus (10, 200, 210, 220, 402, 406) of any of claims 1-3, wherein the housing is at least a part of a watch, bracelet, or ring, the housing further comprising an indication device configured to provide, based on the skin resistance, a visual indicator, an audio indicator, or both.

5. The apparatus (10, 200, 210, 220, 402, 406) of claim 1, further comprising:
a separate housing (504, 514, 524, 560, 630, 640) that is configured to at least partially encircle a second body part of the user such that an inner surface of the separate housing is configured to at least partially contact the second body part, the inner surface comprising an electrically conductive material and the outer surface comprising an electrically conductive material; and
an electrical conductor that extends between at least part of the electrically conductive material of the inner surface of the separate wearable housing (202, 212, 472, 482, 492, 502, 512, 522, 532, 550, 600, 610) and at least part of the electrically conductive material of the outer surface of the separate housing.

6. The apparatus (10, 200, 210, 220, 402, 406) of claim 1, wherein the outer surface of the wearable housing (202, 212, 472, 482, 492, 502, 512, 522, 532, 550, 600, 610) comprises at least one channel (602, 656) that is configured to at least partially contact a second body part of the user.

7. The apparatus (10, 200, 210, 220, 402, 406) of any of claims 1-6, wherein at least part of the outer surface is concentric with at least part of the inner surface.

8. The apparatus (10, 200, 210, 220, 402, 406) of any of claims 1-7, further comprising:
means for receiving, from the circuit, information indicative of the skin resistance between the inner electrode and the outer electrode; and
means for determining a galvanic skin response of the user based, at least in part, on the skin resistance.

9. The apparatus (10, 200, 210, 220, 402, 406) of claim 8, further comprising means for determining that the skin resistance is within a skin contact threshold value, wherein the determination of the galvanic skin response is performed in response to the determination that the skin resistance is within the skin contact threshold value.

10. The apparatus (10, 200, 210, 220, 402, 406) of claim 9, further comprising:
means for determining that the skin resistance has changed to be beyond the skin contact threshold value; and
means for precluding determination of a galvanic skin response of the user in response to the determination that the skin resistance has changed to be beyond the skin contact threshold value.

11. The apparatus (10, 200, 210, 220, 402, 406) of any of claims 8-10, wherein the determination of the galvanic skin response is based, at least in part, on a difference between the skin resistance from a predetermined skin resistance value.

12. The apparatus (10, 200, 210, 220, 402, 406) of any of claims 8-10, wherein the determination of the galvanic skin response is based, at least in part, on variation in the skin resistance over time.

13. The apparatus (10, 200, 210, 220, 402, 406) of any of claims 1-12, further comprising means for performing:
establishment of a communication channel with a separate apparatus; and
causation of sending information indicative of the skin resistance to the separate apparatus by way of the communication channel.

14. The apparatus (10, 200, 210, 220, 402, 406) of any of claims 8-13, wherein the means comprise at least one processor (11) and at least one memory (12), the memory comprising machine-readable instructions, that when executed cause the apparatus to perform the elements of any of claims 9-12, respectively.

## Patentansprüche

1. Vorrichtung (10, 200, 210, 220, 402, 406), umfassend:
ein tragbares Gehäuse (202, 212, 472, 482, 492, 502, 512, 522, 532, 550, 600, 610), das eine Innenfläche (414, 424, 434, 454, 554, 564) und eine Außenfläche (412, 422, 432, 452, 552, 562) umfasst, wobei das tragbare Gehäuse ausgelegt ist, um ein Körperteil eines Anwenders mindestens teilweise zu umschließen, wobei die Innenfläche ausgelegt ist, um mindestens teilweise das Körperteil zu kontaktieren;
eine innere Elektrode (312), die auf mindestens einem Teil der Innenfläche angeordnet ist;
eine äußere Elektrode (310), die auf mindestens einem Teil der Außenfläche angeordnet ist, so dass die äußere Elektrode elektrisch von der inneren Elektrode isoliert ist, wobei die innere und die äußere Elektrode ausgelegt sind, um Hautwiderstandsmessungen zu ermöglichen; und
mindestens eine Schaltung (314),
**dadurch gekennzeichnet, dass**:
die Schaltung (314) ausgelegt ist, um den Hautwiderstand eines Anwenders zwischen der inneren Elektrode und der äußeren Elektrode zu messen.

2. Vorrichtung (10, 200, 210, 220, 402, 406) nach Anspruch 1, wobei die innere Elektrode und die äußere Elektrode so ausgelegt sind, dass ein elektrischer Hautwiderstand, der zwischen der inneren Elektrode und der äußeren Elektrode gemessen wird, über einen elektrischen Pfad (306, 376, 470, 484, 494, 498) gemessen wird, der länger als eine Distanz zwischen der inneren Elektrode und der äußeren Elektrode durch die Vorrichtung hindurch ist.

3. Vorrichtung (10, 200, 210, 220, 402, 406) nach einem der Ansprüche 1 bis 2, des Weiteren umfassend eine Kommunikationseinrichtung (15), wobei die Kommunikationseinrichtung ausgelegt ist, um Informationen, die den Hautwiderstand angeben, an eine separate Vorrichtung zu senden.

4. Vorrichtung (10, 200, 210, 220, 402, 406) nach einem der Ansprüche 1 bis 3, wobei das Gehäuse mindestens ein Teil einer Uhr, eines Armbands oder Rings ist, wobei das Gehäuse des Weiteren eine Angabevorrichtung ist, die ausgelegt ist, um basierend auf dem Hautwiderstand einen visuellen Indikator, einen akustischen Indikator oder beide bereitzustellen.

5. Vorrichtung (10, 200, 210, 220, 402, 406) nach Anspruch 1, des Weiteren umfassend:
ein separates Gehäuse (504, 514, 524, 560, 630, 640), das ausgelegt ist, um ein zweites Körperteil des Anwenders mindestens teilweise zu umschließen, so dass eine Innenfläche des separaten Gehäuses ausgelegt ist, um mindestens teilweise das zweite Körperteil zu kontaktieren, wobei die Innenfläche ein elektrisch leitendes Material umfasst und die Außenfläche ein elektrisch leitendes Material umfasst; und
einen elektrischen Leiter, der sich zwischen mindestens einem Teil des elektrisch leitenden Materials der Innenfläche des separaten tragbaren Gehäuses (202, 212, 472, 482, 492, 502, 512, 522, 532, 550, 600, 610) und mindestens einem Teil des elektrisch leitenden Materials der Außenfläche des separaten Gehäuses erstreckt.

6. Vorrichtung (10, 200, 210, 220, 402, 406) nach Anspruch 1, wobei die Außenfläche des tragbaren Gehäuses (202, 212, 472, 482, 492, 502, 512, 522, 532, 550, 600, 610) mindestens einen Kanal (602, 656) umfasst, der ausgelegt ist, um ein zweites Körperteil des Anwenders mindestens teilweise zu kontaktieren.

7. Vorrichtung (10, 200, 210, 220, 402, 406) nach einem der Ansprüche 1 bis 6, wobei mindestens ein Teil der Außenfläche konzentrisch mit mindestens einem Teil der Innenfläche ist.

8. Vorrichtung (10, 200, 210, 220, 402, 406) nach einem der Ansprüche 1 bis 7, des Weiteren umfassend:
Mittel zum Empfangen von Informationen, die den Hautwiderstand zwischen der inneren Elektrode und der äußeren Elektrode angeben, von der Schaltung; und
Mittel zum Bestimmen einer galvanischen Hautreaktion des Anwenders mindestens teilweise basierend auf dem Hautwiderstand.

9. Vorrichtung (10, 200, 210, 220, 402, 406) nach Anspruch 8, des Weiteren umfassend Mittel zum Bestimmen, dass der Hautwiderstand innerhalb eines Hautkontaktschwellenwerts liegt, wobei das Bestimmen der galvanischen Hautreaktion in Reaktion auf die Bestimmung durchgeführt wird, dass der Hautwiderstand innerhalb des Hautkontaktschwellenwerts liegt.

10. Vorrichtung (10, 200, 210, 220, 402, 406) nach Anspruch 9, des Weiteren umfassend:
Mittel zum Bestimmen, dass der Hautwiderstand sich auf einen Wert jenseits des Hautkontaktschwellenwerts geändert hat; und
Mittel zum Ausschließen der Bestimmung einer galvanischen Hautreaktion des Anwenders in Reaktion auf die Bestimmung, dass sich der Hautwiderstand auf einen Wert geändert hat, der jenseits des Hautkontaktschwellenwerts liegt.

11. Vorrichtung (10, 200, 210, 220, 402, 406) nach einem der Ansprüche 8 bis 10, wobei die Bestimmung der galvanischen Hautreaktion mindestens teilweise auf einer Differenz zwischen dem Hautwiderstand zu einem vorbestimmten Hautwiderstandswert basiert.

12. Vorrichtung (10, 200, 210, 220, 402, 406) nach einem der Ansprüche 8 bis 10, wobei die Bestimmung der galvanischen Hautreaktion mindestens teilweise auf Variation des Hautwiderstands im Zeitverlauf basiert.

13. Vorrichtung (10, 200, 210, 220, 402, 406) nach einem der Ansprüche 1 bis 12, des Weiteren umfassend Mittel zum Durchführen von folgendem:
Aufbauen eines Kommunikationskanals mit einer separaten Vorrichtung; und
Bewirken, dass Informationen, die den Hautwiderstand angeben, mittels des Kommunikationskanals an die separate Vorrichtung gesendet werden.

14. Vorrichtung (10, 200, 210, 220, 402, 406) nach einem der Ansprüche 8 bis 13, wobei das Mittel mindestens einen Prozessor (11) und mindestens einen Speicher (12) umfasst, wobei der Speicher maschinenlesbare Anweisungen umfasst, die bei Ausführung bewirken, dass die Vorrichtung die Elemente gemäß einem der Ansprüche 9 bis 12 durchführt.

## Revendications

1. Appareil (10, 200, 210, 220, 402, 406), comprenant :
un boîtier portable (202, 212, 472, 482, 492, 502, 512, 522, 532, 550, 600, 610) qui comprend une surface interne (414, 424, 434, 454, 554, 564) et une surface externe (412, 422, 432, 452, 552, 562), le boîtier portable étant configuré pour encercler au moins partiellement une partie du corps d'un utilisateur, la surface interne étant configurée pour être au moins partiellement en contact avec la partie du corps ;
une électrode interne (312) disposée sur au moins une partie de la surface interne ;
une électrode externe (310) disposée sur au moins une partie de la surface externe de telle sorte que l'électrode externe est isolée électriquement de l'électrode interne, les électrodes interne et externe étant configurées pour permettre une mesure de résistance cutanée ; et
au moins un circuit (314) ;
**caractérisé en ce que** :
le circuit (314) est configuré pour mesurer la résistance cutanée d'un utilisateur entre l'électrode interne et l'électrode externe.

2. Appareil (10, 200, 210, 220, 402, 406) de la revendication 1, dans lequel l'électrode interne et l'électrode externe sont configurées de telle sorte qu'une résistance électrique cutanée mesurée entre l'électrode interne et l'électrode externe est mesurée sur un chemin électrique (306, 376, 470, 484, 494, 498) qui est plus long qu'une distance entre l'électrode interne et l'électrode externe à travers l'appareil.

3. Appareil (10, 200, 210, 220, 402, 406) de l'une quelconque des revendications 1 et 2, comprenant en outre un dispositif de communication (15), le dispositif de communication étant configuré pour envoyer des informations indicatives de la résistance cutanée à un appareil séparé.

4. Appareil (10, 200, 210, 220, 402, 406) de l'une quelconque des revendications 1 à 3, dans lequel le boîtier constitue au moins une partie d'une montre, d'un bracelet ou d'une bague, le boîtier comprenant en outre un dispositif d'indication configuré pour fournir, sur la base de la résistance cutanée, un indicateur visuel, un indicateur sonore, ou les deux.

5. Appareil (10, 200, 210, 220, 402, 406) de la revendication 1, comprenant en outre :
un boîtier séparé (504, 514, 524, 560, 630, 640) qui est configuré pour encercler au moins partiellement une deuxième partie du corps de l'utilisateur de telle sorte qu'une surface interne du boîtier séparé est configurée pour être au moins partiellement en contact avec la deuxième partie du corps, la surface interne comprenant un matériau électriquement conducteur et la surface externe comprenant un matériau électriquement conducteur ; et
un conducteur électrique qui s'étend entre au moins une partie du matériau électriquement conducteur de la surface interne du boîtier portable séparé (202, 212, 472, 482, 492, 502, 512, 522, 532, 550, 600, 610) et au moins une partie du matériau électriquement conducteur de la surface externe du boîtier séparé.

6. Appareil (10, 200, 210, 220, 402, 406) de la revendication 1, dans lequel la surface externe du boîtier portable (202, 212, 472, 482, 492, 502, 512, 522, 532, 550, 600, 610) comprend au moins un canal (602, 656) qui est configuré pour être au moins partiellement en contact avec une deuxième partie du corps de l'utilisateur.

7. Appareil (10, 200, 210, 220, 402, 406) de l'une quelconque des revendications 1 à 6, dans lequel au moins une partie de la surface externe est concentrique avec au moins une partie de la surface interne.

8. Appareil (10, 200, 210, 220, 402, 406) de l'une quelconque des revendications 1 à 7, comprenant en outre :
des moyens pour recevoir, depuis le circuit, des informations indicatives de la résistance cutanée entre l'électrode interne et l'électrode externe ; et
des moyens pour déterminer une réponse galvanique cutanée de l'utilisateur au moins en partie sur la base de la résistance cutanée.

9. Appareil (10, 200, 210, 220, 402, 406) de la revendication 8, comprenant en outre des moyens pour déterminer que la résistance cutanée se situe en deçà d'une valeur seuil de contact cutané, la détermination de la réponse galvanique cutanée étant effectuée en réponse à la détermination que la résistance cutanée se situe en deçà de la valeur seuil de contact cutané.

10. Appareil (10, 200, 210, 220, 402, 406) de la revendication 9, comprenant en outre :
des moyens pour déterminer que la résistance cutanée a changé pour se situer au-delà de la valeur seuil de contact cutané ; et
des moyens pour empêcher la détermination d'une réponse galvanique cutanée de l'utilisateur en réponse à la détermination que la résistance cutanée a changé pour se situer au-delà de la valeur seuil de contact cutané.

11. Appareil (10, 200, 210, 220, 402, 406) de l'une quelconque des revendications 8 à 10, dans lequel la détermination de la réponse galvanique cutanée est au moins en partie basée sur une différence entre la résistance cutanée et une valeur prédéterminée de résistance cutanée.

12. Appareil (10, 200, 210, 220, 402, 406) de l'une quelconque des revendications 8 à 10, dans lequel la détermination de la réponse galvanique cutanée est au moins en partie basée sur une variation dans le temps de la résistance cutanée.

13. Appareil (10, 200, 210, 220, 402, 406) de l'une quelconque des revendications 1 à 12, comprenant en outre des moyens pour effectuer :
l'établissement d'une voie de communication avec un appareil séparé ; et
l'induction de l'envoi d'informations indicatives de la résistance cutanée à l'appareil séparé au moyen de la voie de communication.

14. Appareil (10, 200, 210, 220, 402, 406) de l'une quelconque des revendications 8 à 13, dans lequel les moyens comprennent au moins un processeur (11) et au moins une mémoire (12), la mémoire comprenant des instructions lisibles par machine qui, lorsqu'elles sont exécutées, conduisent l'appareil à effectuer les éléments de l'une quelconque des revendications 9 à 12, respectivement.
